(19) Europäisches Patentamt
European Patent Office
Office européen des brevets

(11) **EP 4 404 106 A1**

(12) **EUROPEAN PATENT APPLICATION**

(43) Date of publication:
**24.07.2024 Bulletin 2024/30**

(21) Application number: **23218500.9**

(22) Date of filing: **20.12.2023**

(51) International Patent Classification (IPC):
**G06N 5/01** *(2023.01)* **G06N 5/045** *(2023.01)*
**G06N 20/00** *(2019.01)* **G16H 50/20** *(2018.01)*

(52) Cooperative Patent Classification (CPC):
**G06N 5/01; G06N 5/045; G06N 20/00; G16H 50/20; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.01.2023 JP 2023006486**

(71) Applicant: **Hitachi, Ltd.**
**Tokyo 100-8280 (JP)**

(72) Inventors:
• **NAKAMURA, Yasuaki**
  **Tokyo, 100-8280 (JP)**
• **TAKEUCHI, Wataru**
  **Tokyo, 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(54) **ANALYSIS DEVICE, ANALYSIS METHOD, AND ANALYSIS PROGRAM**

(57) An analysis device includes: an acquisition unit that acquires, for a plurality of pieces of data to be analyzed having a value of each factor in a factor group, combined branch conditions obtained by combining a plurality of branch conditions in the factor group and values of the combined branch conditions of each piece of data to be analyzed of the plurality of pieces of data to be analyzed; and a search unit that divides the plurality of pieces of data to be analyzed having the values of the combined branch conditions on the basis of the combined branch conditions and searches for a first decision tree.

## FIG. 20

**Description**

CLAIM OF PRIORITY

The present application claims priority from Japanese patent application No. 2023-6486 filed on January 19, 2023, the content of which is hereby incorporated by reference into this application.

BACKGROUND OF THE INVENTION

1. Field of the Invention

The present invention relates to an analysis device, an analysis method, and an analysis program for analyzing data.

2. Description of the Related Art

Non-Patent Literature 1 discloses a method for estimating heterogeneity of causal effects in experimental and observational studies and conducting hypothesis tests about the magnitude of differences in treatment effects across subsets of the population.

The method of Non-Patent Literature 1 is a data-driven approach to partition data into subpopulations that differ in the magnitude of treatment effects. The approach enables the construction of valid confidence intervals for treatment effects, even with many covariates relative to the sample size, and without "sparsity" assumptions. This approach is an "honest" approach to estimation, whereby one sample is used to construct the partition and another to estimate treatment effects for each subpopulation. This approach builds on regression tree methods, modified to optimize for goodness of fit in treatment effects and to account for honest estimation. A model selection criterion anticipates that bias will be eliminated by honest estimation and also accounts for the effect of making additional splits on the variance of treatment effect estimates within each subpopulation.

CITATION LIST

NON-PATENT LITERATURE

NON-PATENT LITERATURE 1: Athey, Susan, et al, "Recursive partitioning for heterogeneous causal effects" Proceedings of the National Academy of Sciences 113.27 (2016): 7353-7360

SUMMARY OF THE INVENTION

However, in Non-Patent Literature 1, if there are a plurality of branch conditions, the number of samples of the population decreases and learning accuracy decreases, and thus, it is difficult to find complicated branch conditions by deepening the causal tree. Such a problem occurs not only in the medical field but also in other fields, but particularly in the medical field, the population tends to be originally small, and thus, the problem becomes more remarkable.

An object of the present invention is to improve accuracy of estimation using a plurality of branch conditions.

An analysis device according to one aspect of the invention disclosed in the present application includes: an acquisition unit that acquires, for a plurality of pieces of data to be analyzed having a value of each factor in a factor group, combined branch conditions obtained by combining a plurality of branch conditions in the factor group and values of the combined branch conditions of each piece of data to be analyzed of the plurality of pieces of data to be analyzed; and a search unit that divides the plurality of pieces of data to be analyzed having the values of the combined branch conditions on the basis of the combined branch conditions and searches for a first decision tree.

According to a representative embodiment of the present invention, it is possible to improve accuracy of estimation using a plurality of branch conditions. Problems, configurations, and effects other than those described above will be clarified by the following description of embodiments, and the like.

BRIEF DESCRIPTION OF THE DRAWINGS

Fig. 1 is an explanatory diagram indicating an example of outcomes of a prognostic factor and a predictive factor;
Fig. 2 is an explanatory diagram indicating an example in which a patient population is divided with predictive factor within patient characteristics considered to be significantly effective for a treatment effect $\tau$ and weighted at the time of learning;

Fig. 3 is a block diagram illustrating a hardware configuration example of an analysis device;

Fig. 4 is a block diagram illustrating a functional configuration example of the analysis device according to a first embodiment;

Fig. 5 is an explanatory diagram indicating an example of a weight table illustrated in Fig. 4;

Fig. 6 is an explanatory diagram indicating an example of a healthcare DB illustrated in Fig. 4;

Fig. 7 is an explanatory diagram indicating an example of a patient data table according to the first embodiment;

Fig. 8 is an explanatory diagram illustrating an example of an input screen of the analysis device according to the first embodiment;

Fig. 9 is a flowchart indicating an example of analysis processing procedure by the analysis device;

Fig. 10 is an explanatory diagram indicating an example of a stratification result;

Fig. 11 is an explanatory diagram indicating another example of the stratification result;

Fig. 12 is a flowchart indicating an example of detailed processing procedure of stratification processing (step S902) indicated in Fig. 9;

Fig. 13 is a flowchart indicating an example of detailed processing procedure of branch condition search processing (step S1002) indicated in Fig. 10;

Fig. 14 is a box-and-whisker diagram indicating a prediction error improvement rate compared to the rate before division in the method in related art and in the first embodiment;

Fig. 15 is a flowchart indicating an example of processing procedure of generating a weight table by a generation unit according to a second embodiment;

Fig. 16 is a histogram indicating search results from a medical literature database;

Fig. 17 is a flowchart indicating an example of processing procedure of generating a weight table according to a third embodiment;

Fig. 18 is a block diagram illustrating a functional configuration example of an analysis device according to a fourth embodiment;

Fig. 19 is an explanatory diagram indicating an example of a patient data table according to the fourth embodiment;

Fig. 20 is an explanatory diagram indicating an example of a causal tree generated by relearning;

Fig. 21 is an explanatory diagram illustrating an example of an input screen of the analysis device according to the first embodiment; and

Fig. 22 is a flowchart indicating an example of processing procedure of generating combined branch conditions by a generation unit according to the fourth embodiment.

DESCRIPTION OF THE PREFERRED EMBODIMENTS

<Outcomes of prognostic factor and predictive factor>

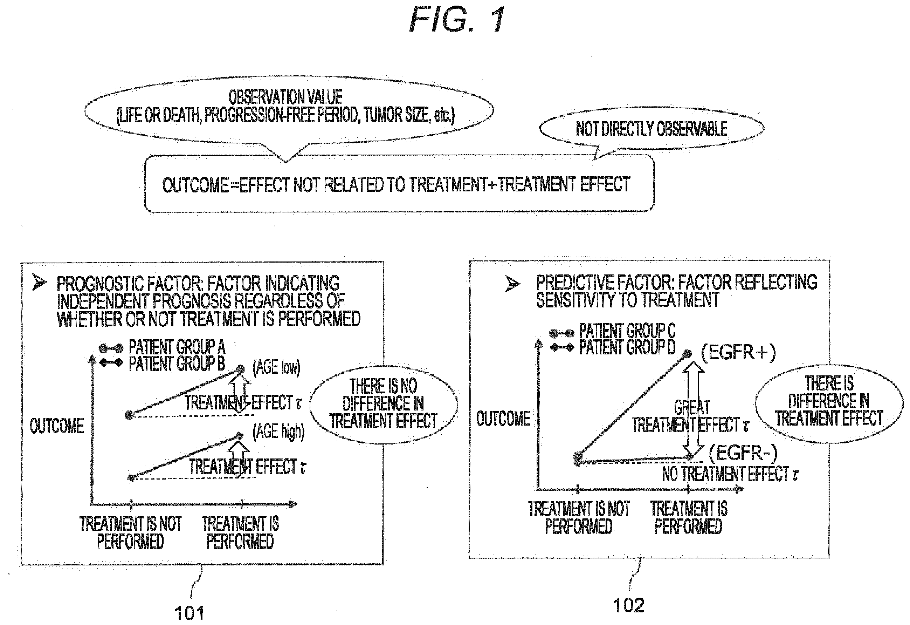

Fig. 1 is an explanatory diagram indicating an example of outcomes of a prognostic factor and a predictive factor. The outcome is, for example, an observation value such as life or death, a progression-free period, or a tumor size, and is a value in which an effect not related to treatment and a treatment effect are inherent. The effect not related to treatment and the treatment effect, respectively, are not directly observable.

A graph 101 indicates outcomes before and after treatment of patient groups A and B, where the patient population was grouped in accordance with the presence or absence of a prognostic factor. A graph 102 indicates outcomes before and after treatment of patient groups C and D, where the patient population was grouped in accordance with the presence or absence of a predictive factor.

Each of the prognostic factor and the predictive factor.is any of factors of a factor group constituting characteristics (hereinafter, patient characteristics) possessed by the patient and is a quantitative variable changed by an outcome, that is, a covariate. The prognostic factor is a factor that indicates an independent prognosis regardless of whether or not treatment is performed, for example, the age of the patient. The predictive factor is a factor reflecting sensitivity to treatment, for example, epidermal growth factor receptor (EGFR) and is a factor indicating a treatment effect different depending on the presence or absence of the predictive factor.

In the graph 101, the patient group A is a set of patients (age: low) having a low value of the prognostic factor indicating the age, and the patient group B is a set of patients (age: high) having a higher value of the prognostic factor indicating the age than that of the patient group A. In the graph 101, the outcome before and after treatment varies depending on the difference between the patient groups A and B, but there is no difference in a treatment effect $\tau$ (difference in the outcome before and after treatment) between the patient groups A and B.

In the graph 102, the patient group C is a set of patients (EGFR+) having a high value of the predictive factor indicating EGFR, and the patient group D is a set of patients (EGFR-) having a lower value of the predictive factor indicating EGFR than that of the patient group C. In the graph 102, the outcome before and after treatment varies depending on the difference between the patient groups C and D, and there is also a difference in the treatment effect $\tau$ (difference in the outcome before and after treatment) between the patient groups C and D. In the graph 102, the treatment effect $\tau$ of the patient group C is greater than the treatment effect $\tau$ of the patient group D.

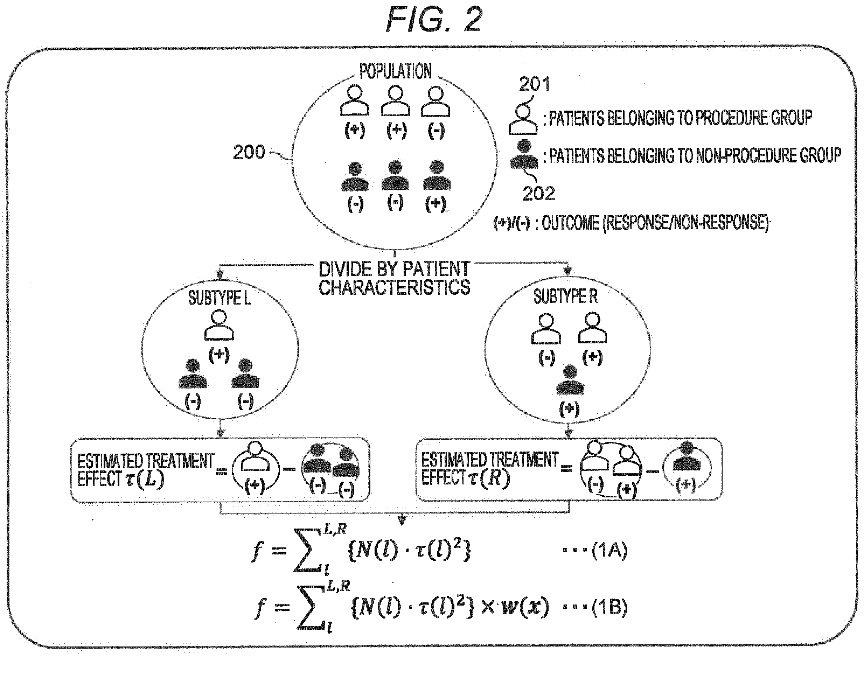

In this manner, by stratifying the patient population with a predictive factor such as EGFR, it is possible to support treatment selection through classification of the condition for each treatment effect $\tau$. In a case where the patient population is not stratified with the predictive factor, it is possible to predict the treatment effect $\tau$ by a method as indicated in Fig. 2.

Fig. 2 is an explanatory diagram indicating an example in which the patient population is divided with the predictive factor within patient characteristics considered to be significantly effective for the treatment effect $\tau$ and weighted at the time of learning. In the population 200, there are patients 201 belonging to a procedure group and patients 202 belonging to a non-procedure group. The procedure group is a set of patients for whom procedure for a sick/injured area has been performed, and the non-procedure group is a set of patients for whom procedure for a sick/injured area has not been performed. In addition, (+) indicates a response, and (-) indicates a non-response. Hereinafter, the patients 201 and 202 who achieved a response are referred to as patients 201(+) and 202(+), respectively, and the patients 201 and 202 who did not achieve a response are referred to as patients 201(-) and 202(-), respectively.

In other words, the patient 201(+) is a patient 201 whose sick/injured is cured by procedure, and the patient 201(-) is a patient 201 whose sick/injured is not cured even by procedure. In addition, the patient 202(+) is a patient 202 whose sick/injured has been cured although procedure has not been performed, and the patient 202(-) is a patient 202 whose sick/injured has not been cured because procedure has not been performed. In Fig. 2, a set of six patients 201 and 202 is set as a population 200 for simplification of description.

Here, the analysis device divides the population 200 of the patients into two groups with a predictive factor x within the patient characteristics that are considered to have a significant effect on the treatment effect $\tau$. One group is referred to as a subtype L, and the other group is referred to as a subtype R.

The estimated treatment effect $\tau(L)$ of the subtype L is a difference between the outcome of the patient 201(+) within the subtype L and the outcome of the patient 202(-) within the subtype L and corresponds to a difference in the treatment effect $\tau$ between the patient groups C and D in Fig. 1.

The estimated treatment effect $\tau(R)$ of the subtype R is a difference between the outcome of the patients 201(+) and 201(-) in the subtype R and the outcome of the patients 202(+) in the subtype R and corresponds to a difference in the treatment effect $\tau$ between the patient groups C and D in Fig. 1.

The analysis device learns a loss function f by a sum of squares (following expression (1A)) of the estimated treatment effects $\tau(L)$ and $\tau(R)$ and predicts the treatment effect $\tau$ of the patient to be predicted by the loss function f.

[Math. 1]

$$f = \sum_{l}^{L,R} \{N(l) \cdot \tau(l)^2\} \qquad \cdots (1A)$$

Note that 1 is an index indicating which treatment effect $\tau(l)$ of the subtypes L and R is achieved. N(l) is the number of samples of the subtype L.

In addition, as described above, by stratifying the patient population with the predictive factor such as EGFR, it is possible to support treatment selection through classification of the condition for each treatment effect $\tau$. On the other hand, in a case where stratification is not performed with the predictive factor, prediction accuracy of the treatment effect $\tau$ decreases. It is therefore also possible to improve the prediction accuracy of the treatment effect $\tau$ by specifying the predictive factor in the patient characteristics that is considered to be significantly effective for the treatment effect $\tau$ in advance and weighting the predictive factor at the time of learning.

In this case, the analysis device applies a weight w(x) related to the predictive factor x obtained by dividing the population 200 into the subtypes L and R to a sum of squares of the estimated treatment effects $\tau(L)$ and $\tau(R)$, thereby learning the loss function f using the following expression (1B) or predicting the treatment effect $\tau$ of the patient to be predicted using the loss function f.

[Math. 2]

$$f = \sum_{l}^{L,R} \{N(l) \cdot \tau(l)^2\} \times w(x) \quad \cdots (1B)$$

Hereinafter, details of the analysis device illustrated in Figs. 1 and 2 will be described as first to fourth embodiments. If values of the weight w(x) in the above expression (1B) are all 1 regardless of the prognostic factor and the predictive factor, the above expression (1B) is the same expression as the above expression (1A). Thus, the following embodiments will be described on the basis of the above expression (1B). Of course, it is needless to say that a form in which the value of the weight w(x) is set to 1, that is, a form in which no weight is added is also an object of the present invention.

First embodiment

In the first embodiment, an analysis device in a case where the weight w(x) is specified in advance will be described. Further, the present invention is not limited by the following examples.

<Hardware configuration example of analysis device>

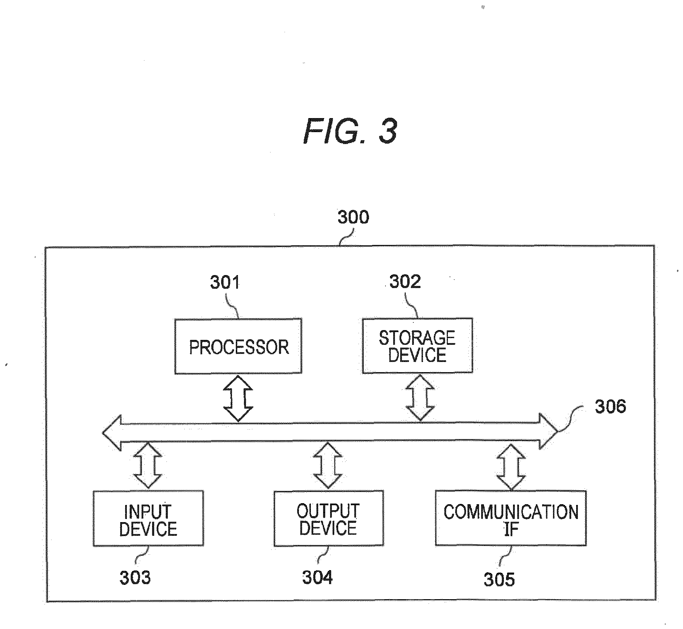

Fig. 3 is a block diagram illustrating a hardware configuration example of the analysis device. An analysis device 300 includes a processor 301, a storage device 302, an input device 303, an output device 304, and a communication interface (communication IF) 305. The processor 301, the storage device 302, the input device 303, the output device 304, and the communication IF 305 are connected by a bus 306. The processor 301 controls the analysis device 300. The storage device 302 is a work area of the processor 301. The storage device 302 is a non-transitory or transitory recording medium that stores various kinds of programs and data. Examples of the storage device 302 include a read only memory (ROM), a random access memory (RAM), a hard disk drive (HDD), and a flash memory. The input device 303 inputs data. Examples of the input device 303 include a keyboard, a mouse, a touch panel, a numeric keypad, a scanner, a microphone, and a sensor. The output device 304 outputs data. Examples of the output device 304 include a display, a printer, and a speaker. The communication IF 305 is connected to a network and transmits and receives data.

<Functional configuration example of analysis device>

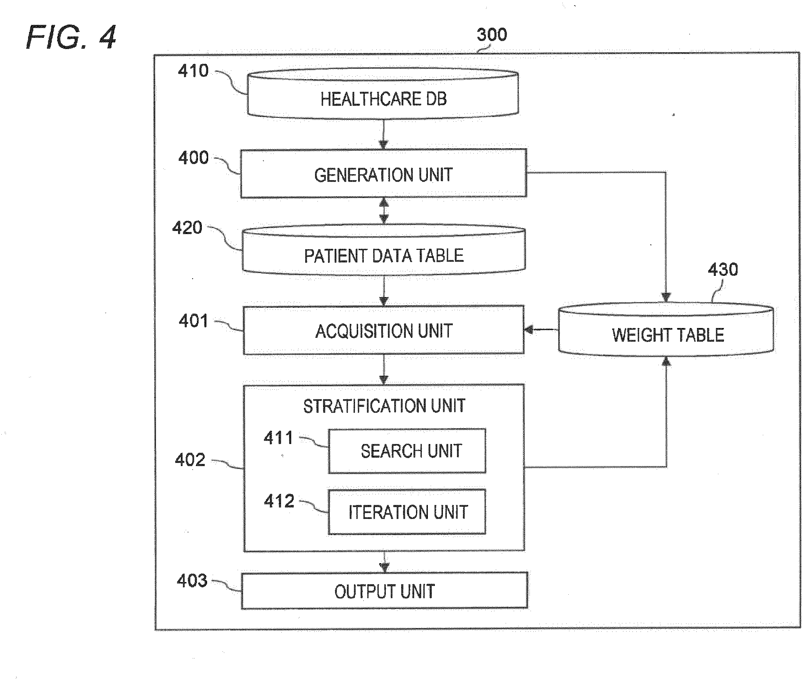

Fig. 4 is a block diagram illustrating a functional configuration example of the analysis device according to the first embodiment. The analysis device 300 includes a generation unit 400, an acquisition unit 401, a stratification unit 402, an output unit 403, a healthcare DB 410, and a patient data table 420. Specifically, the healthcare DB 410 and the patient data table 420. have, for example, data structures stored in the storage device 302 illustrated in Fig. 3 and can be accessed by the processor 301. Specifically, the generation unit 400, the acquisition unit 401, the stratification unit 402, and the output unit 403 are functions to be implemented by causing the processor 301 to execute a program stored in the storage device 302 illustrated in Fig. 3, for example.

The generation unit 400 generates the patient data table 420 with reference to the healthcare DB 410. The acquisition unit 401 acquires a plurality of pieces of patient data for specifying patients from the patient data table 420and acquires a weight from the weight table 430. The stratification unit 402 stratifies the patients acquired as the patient data by the acquisition unit 401. The stratification unit 402 includes a search unit 411 and an iteration unit 412. The search unit 411 searches for branch conditions for stratifying the patients. The iteration unit 412 repeatedly executes search for the branch conditions by the search unit 411 and division of the patients using the branch conditions. The output unit 403 outputs the stratification result by the stratification unit 402.

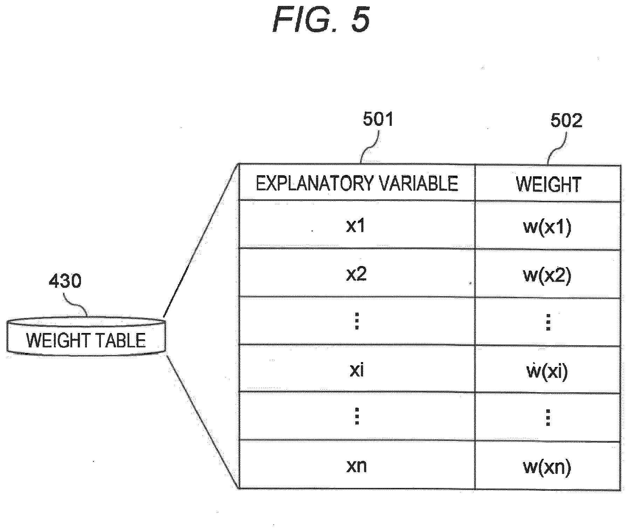

Fig. 5 is an explanatory diagram indicating an example of the weight table 430 illustrated in Fig. 4. The weight table 430 has an explanatory variable 501 and a weight 502 as fields. A combination of a value of the explanatory variable 501 and a value of the weight 502 in the same row is an entry for specifying one explanatory variable 501.

As described above, the explanatory variable 501 is a field for specifying a factor reflecting sensitivity to treatment and holds x1, x2,..., xi,..., xn (n is an integer of 1 or more, and i is an integer satisfying $1 \leq i \leq n$) as identification information for uniquely specifying a predictive factor from a number of explanatory variables. Hereinafter, the value of the explanatory variable 501 may be referred to as a predictive factor $x_i$. The weight 502 is an index value indicating significance of the treatment effect $\tau$ and is input to the above expression (1). In this example, as the value of the weight 502 is larger, prediction accuracy of the treatment effect $\tau$ is improved.

Note that, in the first embodiment, the weight table 430 is prepared in advance. The analysis device 300 can add, change, or delete an entry of the weight table 430 and change the value of the weight 502 through user operation.

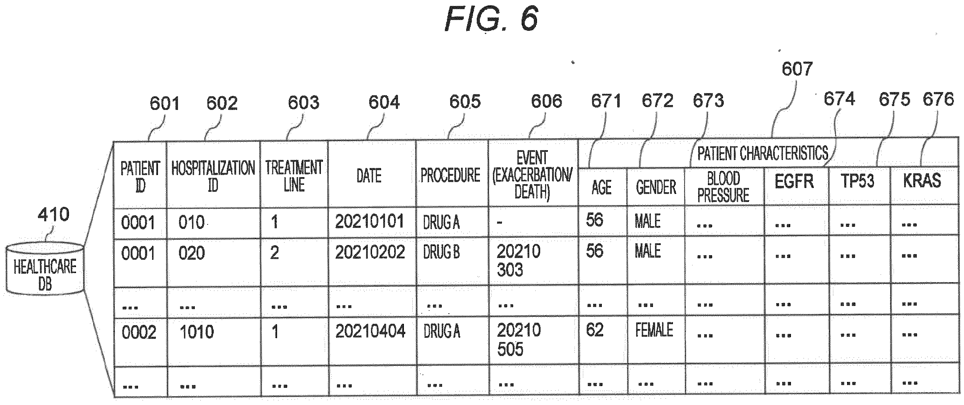

Fig. 6 is an explanatory diagram indicating an example of the healthcare DB 410 illustrated in Fig. 4. The healthcare DB 410 includes as fields, a patient ID 601, a hospitalization ID 602, a treatment line 603, a date 604, a procedure 605, an event 606, and patient characteristics 607. A combination of values of the fields in the same row is an entry that defines one piece of healthcare information. There are one or more entries for one patient. For example, if a patient is hospitalized three times, there are three entries for that patient. Note that, in Fig. 6, healthcare information regarding a sick/injured area to be analyzed (for example, cancer) is defined.

The patient ID 601 is identification information that uniquely identifies the patient. The hospitalization ID 602 is identification information assigned when the patient specified by the patient ID 601 is hospitalized. The treatment line 603 is a number indicating the order of treatment.

The treatment line 603 is a number indicating the order of treatment by administration of an anticancer agent in treatment for cancer. For example, in a case where an anticancer agent is administered for the first time to a certain carcinoma, a value of the treatment line 603 is "1" because it is the first treatment, "2" in a case of the second treatment, "3" in a case of the third treatment,....

The date 604 is the year, month, and date when the treatment by the treatment line 603 was performed. The procedure 605 is content of treatment by the treatment line 603. The event 606 is a result of performing the procedure 605 by the treatment line 603 (for example, exacerbation, death, etc.).

The patient characteristics 607 are explanatory variables indicating a factor group that becomes a feature amount at the time point of the date 604 of the patient specified by the patient ID 601 and includes a covariate. Specifically, the patient characteristics 607 are clinical test values or presence or absence of a genetic mutation, and include, for example, age 671, gender 672, a blood pressure 673, EGFR 674, TP53 675, and KRAS 676 as factors.

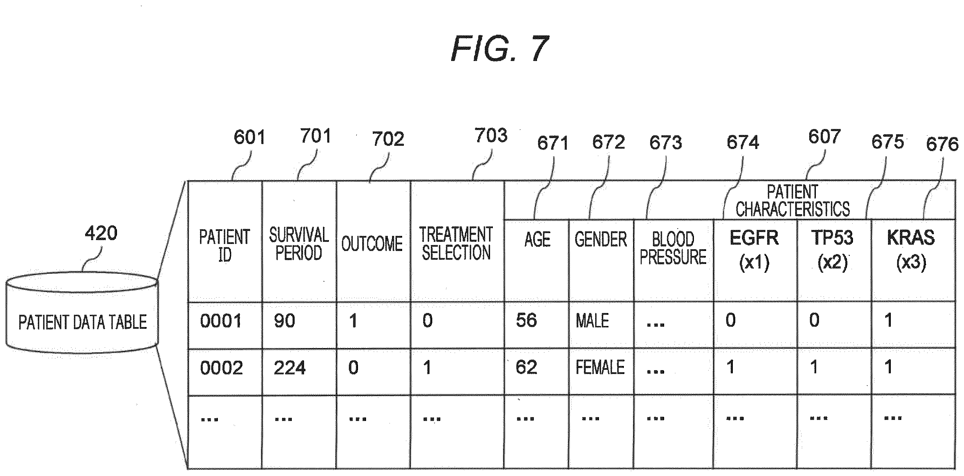

Fig. 7 is an explanatory diagram indicating an example of the patient data table according to the first embodiment. The patient data table 420 is generated by the generation unit 400 with reference to the healthcare DB 410. Note that the patient data table 420 may be stored in the storage device 302 in advance.

The patient data table 420 is a table in which the healthcare DB 410 is collected in units of patients and has, for example, a patient ID 601, a survival period 701, an outcome 702, treatment selection 703, and patient characteristics 607 as fields. A combination of values of the fields in the same row is an entry that defines patient data of one patient.

Note that, in a case where there are a plurality of entries for one patient in the healthcare DB 410, for example, an entry having a maximum value of the treatment line 603 is used for the entry of the patient data table 420.

The survival period 701 is the number of days from the date 604 of the patient specified by the patient ID 601 to the date of death which is the value of the event 606. If there is no value in the event 606, the number of days until the current date is indicated.

The outcome 702 is, for example, an observation value such as life or death, a progression-free period, and a tumor size and is a value in which an effect not related to treatment and a treatment effect are inherent. Here, in the example of Fig. 7, the value of the outcome 702 is a numerical value identifying life and death. For example, "1" indicates survival, and "0" indicates death. The analysis device 300 refers to the event 606 and stores "1" if there is no value in the event 606 and stores "0" if there is the death date in the event 606.

The treatment selection 703 is a value indicating whether or not the patient specified by the patient ID 601 has selected the treatment, and "1" indicates that the patient has selected the treatment, and "0" indicates that the patient has not selected the treatment. The analysis device 300 refers to the procedure 605 and stores "0" if there is no value in the procedure 605 and "1" if there is a value in the procedure 605.

In the following description, for convenience of explanation, the EGFR 674 may be referred to as a factor $x1$, the TP53 675 may be referred to as a factor $x2$, and the KRAS 676 may be referred to as a factor $x3$.

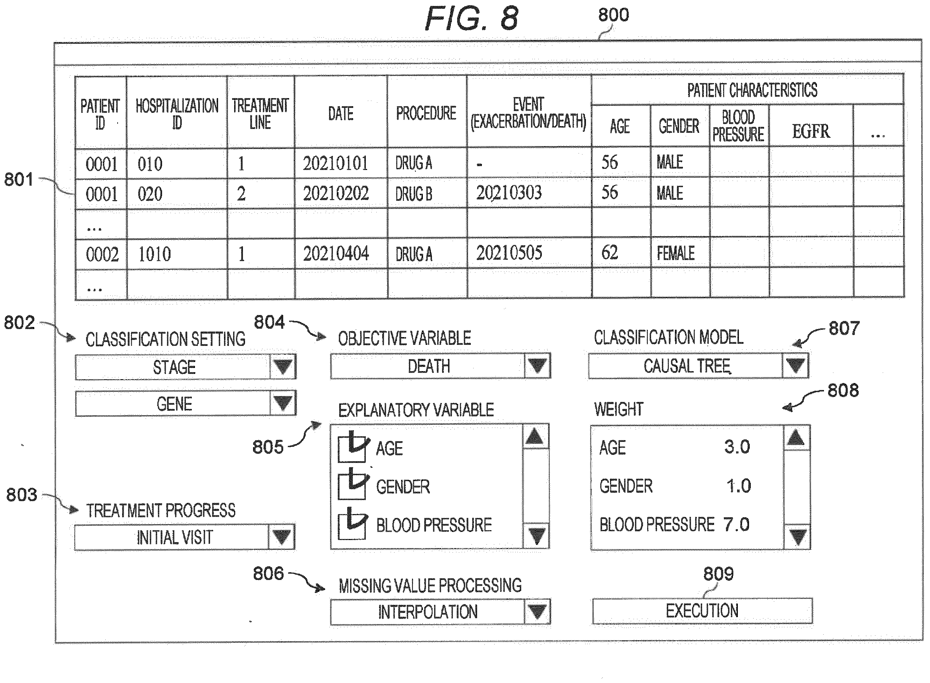

Fig. 8 is an explanatory diagram illustrating an example of an input screen of the analysis device 300 according to the first embodiment. An input screen 800 is displayed on a display device that is an example of the output device 304 of the analysis device 300 or a display device of another computer that can communicate with the analysis device 300 via the communication IF 305. Furthermore, the user can input information to the input screen 800 by operating the input device 303 of the analysis device 300 or an input device of another computer.

The input screen 800 includes a healthcare information setting item 801, a classification setting item 802, a treatment progress item 803, an objective variable item 804, an explanatory variable item 805, a missing value processing item 806, a classification model item 807, a weight item 808, and an execution button 809.

The healthcare information setting item 801 is a user interface that allows selection of a prediction target entry from the entries of the healthcare DB 410 indicated in Fig. 6. The classification setting item 802 is a user interface that allows selection of an item for classifying entries of the healthcare information setting item 801 by classification information such as a cancer stage or a gene of a patient. This makes it possible to narrow down the entries of the healthcare information setting item 801. The treatment progress item 803 is a user interface that allows selection of the treatment line 603 of the patient.

The objective variable item 804 is a user interface that allows selection of an objective variable output from a classification model f. As the objective variable, for example, the event 606 or the procedure 605 of the prediction target patient can be selected. The explanatory variable item 805 is a user interface that allows selection of a factor of the patient characteristics 607 that become one or more explanatory variables of the prediction target patient. In the example of Fig. 8, the age 671, the gender 672, and the blood pressure 673 are selected by inputting a check mark.

The missing value processing item 806 is a user interface that allows selection of missing value processing of the explanatory variable. In the example of Fig. 8, "interpolation" is selected as the missing value processing. The classification model item 807 is a user interface that allows selection of the classification model f. In the example of Fig.

8, a causal tree is selected as the classification model f. The causal tree is a kind of decision tree and is a kind of decision tree for calculating a conditional average treatment effect (CATE) in statistical causal inference.

In the weight item 808, the weight 502 of the explanatory variable 501 selected in the explanatory variable item 805 is displayed. The user may remove the selection of the explanatory variable in the explanatory variable item 805 with reference to the weight 502. For example, the weight 502 of the gender 672 is "1.0", which is lower than the other weights 502, and thus, the user may exclude the gender 672 from the explanatory variable item 805. The execution button 809 is a user interface for causing the analysis device 300 to execute analysis processing by being pressed.

<Analysis processing>

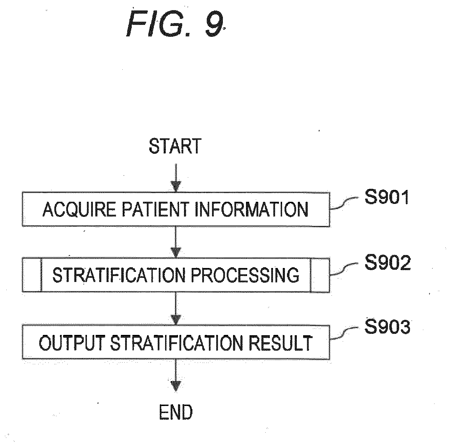

Fig. 9 is a flowchart indicating an example of analysis processing procedure by the analysis device 300. The analysis device 300 generates the patient data table 420 from the healthcare DB 410 if the patient data table 420 is not generated by the generation unit 400. Then, the analysis device 300 causes the acquisition unit 401 to acquire patient data that is an entry from the patient data table 420 (step S901).

Next, the analysis device 300 executes stratification processing by the stratification unit 402 (step S902). The stratification processing (step S902) is processing of stratifying patients using the patient data. Then, the analysis device 300 outputs the stratification result by the stratification processing (step S902) by the output unit 403 (step S903) and ends a series of the analysis processing. In step S903, the analysis device 300 may display the stratification result on a display that is an example of the output device 304, may transmit the stratification result to another computer through the communication IF 305 or may store the stratification result in the storage device 302.

<Stratification result>

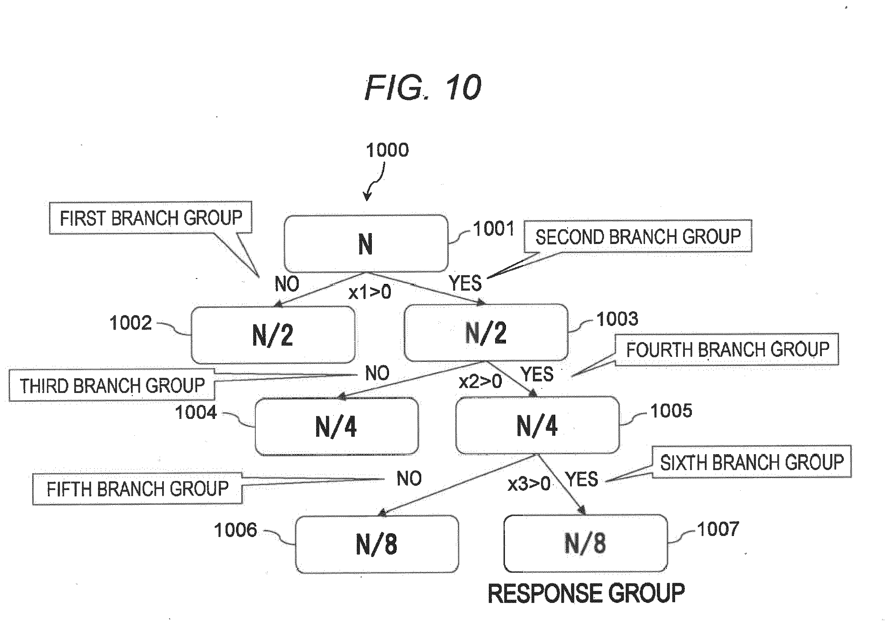

Fig. 10 is an explanatory diagram indicating an example of the stratification result. The stratification result indicated in Fig. 10 is a causal tree 1000 having a tree structure. The causal tree 1000 includes nodes 1001 to 1005. "N" in the nodes 1001 to 1007 is the number of samples, that is, the number of patients. In other words, as an example, a tree structure in which the number of samples is halved by division is indicated.

An analysis target group indicated by the node 1001 is divided into a patient group (node 1003) in which a predictive factor $x1 > 0$ and other patient groups (node 1002). The predictive factor $x1$ and a division threshold "0" for dividing the analysis target group are branch conditions of the node 1001.

The patient group at the node 1003 is divided into a patient group (node 1005) in which a predictive factor $x2 > 0$ and the other patient groups (node 1004). The predictive factor $x2$ and the division threshold "0" for dividing the division target are branch conditions of the node 1003.

The patient group at the node 1005 is divided into a patient group (node 1007) in which a predictive factor $x3 > 0$ and other patient groups (node 1006). The node 1007 that satisfies all branch conditions is a response group. The predictive factor $x3$ and the division threshold "0" for dividing the division target are branch conditions of the node 1005.

No branch condition exists at the nodes 1002, 1004, 1006, and 1007. The causal tree 1000 is constituted with the nodes 1001 to 1007, the connection relationship between the nodes 1001 to 1007, and the branch conditions of the nodes 1001, 1003, and 1005.

Note that Fig. 10 indicates that if the patient group to be divided is divided by the division threshold, the number of patients in the patient group to be divided is halved.

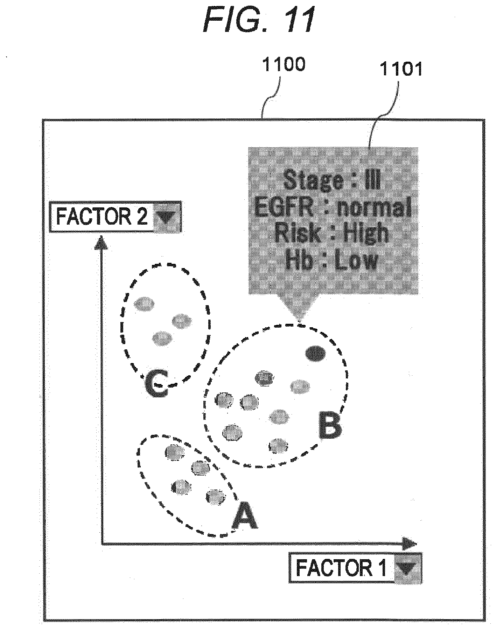

Fig. 11 is an explanatory diagram indicating another example of the stratification result. The stratification result 1100 indicated in Fig. 11 is an example indicated in a graph. The stratification result 1100 is a scatter diagram in which a relationship between the factor 1 and the factor 2 which are covariates, is graphed, and the analysis target group is divided into the patient groups A, B, and C. The covariate is not limited to the combination of the factor 1 and the factor 2, and other combinations can be selected.

Furthermore, in a case where the user operates the input device 303 to designate each of the patient groups A, B, and C, the analysis device 300 may display feature information of the designated patient group. In Fig. 11, in a case where the patient group B is designated, feature information 1101 of the patient group B is displayed.

<Stratification processing>

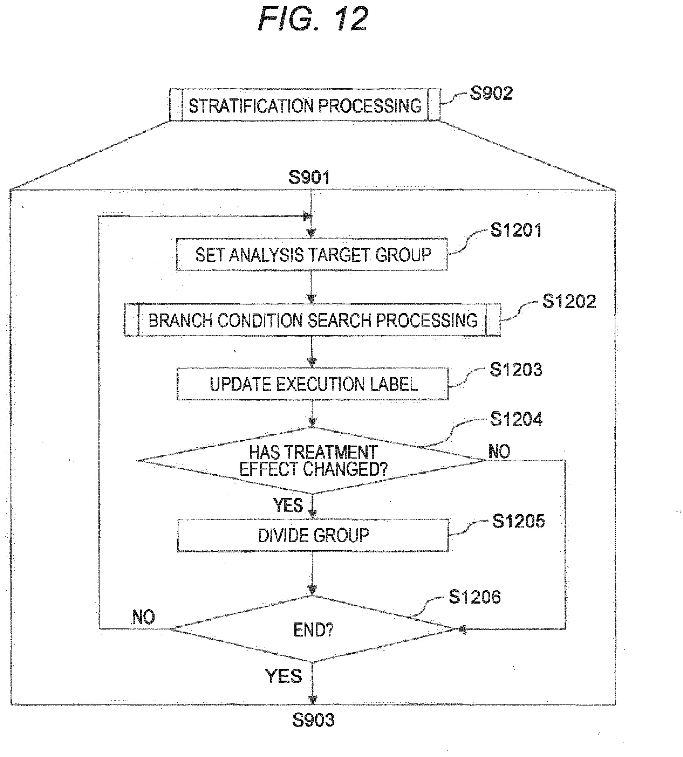

Fig. 12 is a flowchart indicating an example of detailed processing procedure of the stratification processing (step S902) indicated in Fig. 9. The analysis device 300 sets an analysis target group by the iteration unit 412 (step S1201). Specifically, for example, at the time of first execution of step S1201, the analysis device 300 selects an analysis target group for the first execution from the patient data acquired in step S901. The analysis target group at the time of the first execution may be all entries of the patient data or the patient data table 420, may be some patient data corresponding to preset conditions, or may be one or more pieces of patient data.

In addition, at the time of the first execution of step S1201, the analysis device 300 sets an execution label [K, V] in the analysis target group. For example, the execution label [K, V] is a combination of a key K and a value V. At the time of the first execution of step S1201, the key K = 1 and the value V = False are set. False indicates that the branch condition search processing (step S1202) has not been executed, and if the branch condition search processing (step S1202) has been executed, the value V is updated to value V= True indicating that the branch condition search processing (step S1202) has been executed.

Next, the analysis device 300 causes the search unit 411 to execute the branch condition search processing (step S1202). The branch condition search processing (step S1202) is processing of searching for conditions (branch conditions) for branching the analysis target group and generating the causal tree 1000.

Next, the analysis device 300 causes the search unit 411 to update the value V = False of the execution label [K, V] of the analysis target group to a value V = True indicating that the branch condition search processing (step S1202) has been executed (step S1203).

Next, the analysis device 300 determines whether or not the treatment effect has changed before and after the division of the analysis target group by the iteration unit 412 (step S1204). Specifically, for example, the analysis device 300 temporarily divides the analysis target group to be divided under the branch conditions of the causal tree to generate two patient groups (hereinafter, referred to as a first branch group and a second branch group. In addition, in a case where they are not distinguished, they are simply referred to as branch groups.). The analysis device 300 determines which one of the treatment effects of the first branch group and the second branch group has significantly changed with respect to the treatment effect of the analysis target group to be divided.

For example, the analysis device 300 calculates a standard deviation obtained by synthesizing a difference (hereinafter, a first difference) in the treatment effect obtained by comparing the first branch group with the analysis target group and a difference (hereinafter, a second difference) in the treatment effect obtained by comparing the second branch group with the analysis target group. Then, the analysis device 300 determines whether or not at least one of the first difference and the second difference is larger than the standard deviation.

It is determined that the treatment effect has changed from the analysis target group before the division in the branch group that becomes a comparison source, for which the difference is larger than the standard deviation. If at least one of the first difference and the second difference is larger than the standard deviation, it is determined that the treatment effect has changed (step S1204: Yes), and the processing proceeds to step S1205. If both of the first difference and the second difference are equal to or smaller than the standard deviation, the processing proceeds to step S1206.

In a case where the loss function is not improved in the branch condition search processing (step S1202) (that is, in a case where None is returned as the branch condition search result), the analysis device 300 determines that there is no change in the treatment effect (step S1204: No), and the processing proceeds to step S1206.

After step S1204: Yes, the analysis device 300 divides the analysis target group according to the branch conditions used in the temporal division in step S1204 (step S1205). Specifically, for example, the analysis device 300 divides the analysis target group at the parent node in step S1205 of the first time and, if the processing is looped as a result of step S1206: No, divides the analysis target group at the child node of the branch destination in step S1205 of the next time.

In addition, the analysis device 300 gives the execution label to each of the two groups divided in step S1205, that is, the first branch group and the second branch group. Specifically, for example, the analysis device 300 duplicates the execution label [K, V] of the analysis target group for each of the first branch group and the second branch group. Then, the analysis device 300 assigns a branch number "1" to the end of the key K of the execution label [K, V] of the first branch group and updates the value V from V = True to V = False. Similarly, the analysis device 300 assigns a branch number "2" to the end of the key K of the execution label [K, V] of the second branch group and updates the value V from V = True to V = False.

For example, if the execution label [K, V] of the analysis target group is [1, True], the execution label [K, V] of the first branch group is [11, False], and the execution label [K, V] of the second branch group is [12, False], Then, the processing proceeds to step S1206.

The analysis device 300 determines whether or not end conditions are satisfied (step S1206). The end conditions are, for example, the number of executions (that is, the depth of the branch) of preset group division (step S1205) or a lower limit of the number of samples in the group. Specifically, for example, in a case where the number of executions of the group division (step S1205) does not reach a predetermined number of times or more, it is determined that the end conditions are not satisfied (step S1206: No), and the processing returns to step S1201. On the other hand, in a case where the number of executions of the group division (step S1205) reaches the predetermined number of times or more, the value V of each of the first branch group and the second branch group is updated from V = False to V = True, and it is determined that the end conditions are satisfied (step S1206: Yes), the stratification processing (step S902) ends, and the processing proceeds to step S903.

In a case where the end conditions are the lower limit of the number of samples in the group, the analysis device 300 determines whether or not the group is divided by execution of group division (step S1205), and the number of samples of each of the first branch group and the second branch group is below the lower limit of the number of samples in the group. In a case where at least one of the number of samples of the first branch group and the second branch group is below the lower limit of the number of samples in the group, it is determined that the end conditions are not satisfied (step S1206: No), and the processing returns to step S1201. On the other hand, in a case where both the numbers of samples of the first branch group and the second branch group are equal to or larger than the lower limit of the number of samples in the group, the value V of each of the first branch group and the second branch group is updated from V = False to V = True, it is determined that the end conditions are satisfied (step S1206: Yes), the stratification processing (step S902) ends, and the processing proceeds to step S903.

In a case where the treatment effect has not changed (step S1204: No), the analysis device 300 determines whether or not the number of samples in the analysis target group is below the lower limit of the number of samples in the group. In a case where the analysis target group is below the lower limit of the number of samples in the group, it is determined that the end conditions are not satisfied (step S1206: No), and the processing returns to step S1201. On the other hand, in a case where the number of samples of the analysis target group is equal to or larger than the lower limit of the number of samples in the group, the value V of each of the first branch group and the second branch group is updated from V = False to V = True, and it is determined that the end conditions are satisfied (step S1206: Yes), the stratification processing (step S902) ends, and the processing proceeds to step S903.

In other words, in a case where there is a group in which the value V of the execution label [K, V] is "False", it is determined that the end conditions are not satisfied (step S1206: No), and the processing returns to step S1201.

In a case where the processing returns to step S1201 as a result of step S1206: No, the analysis device 300 sets a group in which the value of the execution label [K, V] is "False" as the next analysis target group (step S1201) and similarly executes steps S1202 to S1206.

In the example of the group division (step S1205) described above, the execution label [K, V] of the first branch group is [11, False], and the execution label [K, V] of the second branch group is [12, False]. Thus, the first branch group and the second branch group are set as analysis target groups (step S1201), and steps S1202 to S1206 are executed for each analysis target group.

Here, the causal tree 1000 illustrated in Fig. 10 will be specifically described as an example. First, at the time of the first execution, the analysis device 300 temporarily divides the analysis target group into a first branch group (x1 > 0: Yes) and a second branch group (x1 > 0: No) under the branch conditions (x1 > 0) of the node 1001. Here, it is assumed that the treatment effect has changed for either the first branch group (x1 > 0: Yes) or the second branch group (x1 > 0: No) (step S1204: Yes). As a result, the analysis device 300 divides the analysis target group into the first branch group (x1 > 0: No) and the second branch group (x1 > 0: Yes) under the branch conditions (x1 > 0) of the node 1001 (step S1205).

In addition, the analysis device 300 generates the execution label [11, False] of the first branch group (x1 > 0: No) and the execution label [12, False] of the second branch group (x1 > 0: Yes) by using the execution label [1, True] of the analysis target group.

The first branch group (x1 > 0: No) transitions to the node 1002. There are no branch conditions at the node 1002, and thus, the analysis device 300 ends search for the first branch group (x1 > 0: No) (step S1206: Yes) and updates the execution label [11, False] to the execution label [11, True].

The execution label of the second branch group (x1 > 0: Yes) is [12, False], and the value V is False. Thus, the analysis device 300 sets the second branch group (x1 > 0.: Yes) as the next analysis target group (step S1206: No → S1201).

The analysis device 300 specifies the node 1003 to which the analysis target group (x1 > 0: Yes) transitions in the causal tree 1000 and updates the execution label [12, False] to the execution label [12, True].

Then, the analysis device 300 temporarily divides the analysis target group (x1 > 0: Yes) into a third branch group (x2 > 0: No) and a fourth branch group (x2 > 0: Yes) under the branch conditions (x2 > 0). Here, it is assumed that the treatment effect has changed for either the third branch group (x2 > 0: No) or the fourth branch group (x2 > 0: Yes) (step S1204: Yes). The analysis device 300 divides the analysis target group (x1 > 0: No) into the third branch group (x2 > 0: No) and the fourth branch group (x2 > 0: Yes) under the branch conditions (x2 > 0) (step S1205).

In addition, the analysis device 300 uses the execution label [12, True] of the analysis target group (x1 > 0: Yes) to generate the execution label [123, False] of the third branch group (x2 > 0: No) and the execution label [124, False] of the fourth branch group (x2 > 0: Yes).

The third branch group (x2 > 0: No) transitions to the node 1004. There are no branch conditions at the node 1004, and thus, the analysis device 300 ends search for the third branch group (x2 > 0: No) (step S1206: Yes) and updates the execution label [123, False] to the execution label [123, True].

The execution label of the fourth branch group (x2 > 0: Yes) is [124, False] and the value V is False. Thus, the analysis device 300 sets the fourth branch group (x2 > 0: Yes) as the next analysis target group (step S1206: No → S1201).

The analysis device 300 specifies the node 1005 to which the analysis target group (x2 > 0: Yes) transitions in the causal tree 1000 and updates the execution label [124, False] to the execution label [124, True].

Then, the analysis device 300 temporarily divides the analysis target group (x2 > 0: Yes) into a fifth branch group (x3 > 0: No) and a sixth branch group (x3 > 0: Yes) under the branch conditions (x3. > 0). Here, it is assumed that the treatment effect has changed for either the fifth branch group (x3 > 0: No) or the sixth branch group (x3 > 0: Yes) (step S1204: Yes). The analysis device 300 divides the analysis target group (x2 > 0: No) into the fifth branch group (x3 > 0: No) and the sixth branch group (x3 > 0: Yes) under the branch conditions (x3 > 0) (step S1205) .

In addition, the analysis device 300 uses the execution label [124, True] of the analysis target group (x2 > 0: Yes) to generate the execution label [1245, False] of the fifth branch group (x3 > 0: No) and the execution label [1246, False] of the sixth branch group (x3 > 0: Yes) .

The fifth branch group (x3 > 0: No) transitions to the node 1006. There are no branch conditions at the node 1006, and thus, the analysis device 300 ends search for the fifth branch group (x3 > 0: No) (step S1206: Yes) and updates the execution label [1245, False] to the execution label [1245, True] .

Similarly, the sixth branch group (x3 > 0: Yes) transitions to the node 1007. There are no branch conditions at the node 1007, and thus, the analysis device 300 ends search for the sixth branch group (x3 > 0: Yes) (step S1206: Yes) and updates the execution label [1246, False] to the execution label [1246, True].

Then, the analysis device 300 outputs the execution labels generated so far, the groups corresponding to the execution labels, and the branch conditions used for division as the stratification results.

Note that, in step S903 in Fig. 9, the analysis device 300 outputs, for example, the causal tree which has a tree structure from an initial analysis target group to a branch group at a terminal as the stratification results by the output unit 403. In this event, the execution labels of the respective groups of the stratification results may be reassigned to have ascending order numbers starting from 0 with the initial analysis target group as the start position.

As described above, in the stratification processing (step S902), search that maximizes the treatment effect is executed for each branch group generated in the branch, and stratification that maximizes the treatment effect is implemented.

<Branch condition search processing (step S1002)>

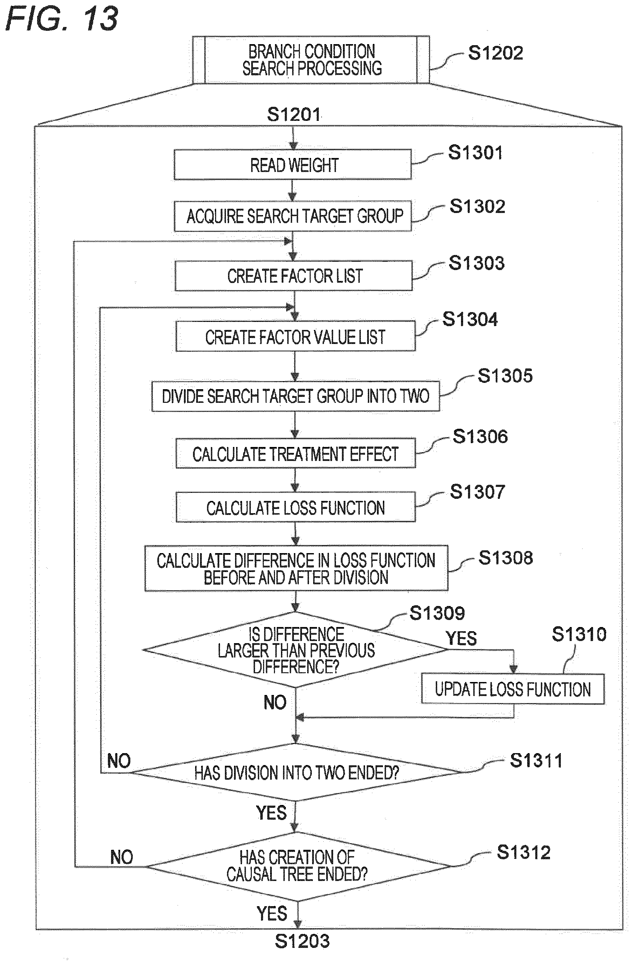

Fig. 13 is a flowchart indicating an example of detailed processing procedure of the branch condition search processing (step S1002) indicated in Fig. 10. The search unit 411 reads the weight 502 of the explanatory variable 501 from the weight table 430 (step S1301).

Next, the search unit 411 acquires a search target group from the analysis target group (step S1302) . Specifically, for example, the search unit 411 may directly use the analysis target group as the search target group_or may divide the analysis target group into training data and verification data. In a case of division, the training data becomes the search target group, and the verification data is used to estimate the treatment effect (step S1306).

Next, the search unit 411 randomly selects factors that are covariates in the search target group, creates a list of the selected factors (factor list) (step S1303) and creates a list of values of the selected factors (factor value list) (step S1304). The factor list is a list of fields indicating factors serving as covariates such as the age 671, the blood pressure 673, and the EGFR 674. The factor group selected in the factor list has less factors than all factors. The causal tree is created for each factor list.

The factor value list is a list including values (56 [years old], 62 [years old],..., 90 [ml], 127 [ml],...) of the selected factors such as the age 671, the blood pressure 673, and the EGFR 674.

In addition, in step S1304, the search unit 411 specifies a preset predictive factor from the factor list and extracts a value of the specified predictive factor (hereinafter, the search target predictive factor) from the factor value list.

In steps S1301, S1303, and S1304, the search unit 411 selects an unselected predictive factor and its weight.

Next, the search unit 411 divides the search target group into two using the search target predictive factor (step S1305). This data division is processing of dividing the search target group into subtypes L and R according to the patient characteristics illustrated in Fig. 2. Every time the processing returns from steps S1311 and S1312, a different predictive factor is selected as the search target predictive factor. As in Fig. 2, one of the divided groups is referred to as the subtype L, and the other group is referred to as the subtype R.

Next, the search unit 411 calculates the treatment effect $\tau$ for each of the subtypes L and R (step S1306). The treatment effect $\tau$ is calculated by the following expression (2).

$$\tau (1) = E [Y|T = 1] - E [Y|T = 0] \cdots (2)$$

In a case of the subtype L, 1 = L, and in a case of the subtype R, 1 = R. Y is an outcome (for example, the event 606). T is a binary variable indicating treatment selection, T = 1 indicating that the treatment has been selected (the procedure 605 has been performed) and T = 0 indicating that the treatment has not been selected (the procedure 605 has not been performed). Further, E[] is an expected value calculation operator. E[] is, for example, a sum of outcomes Y. The treatment effects $\tau(L)$ and $\tau(R)$, which are the second treatment effects, are calculated by the above expression (2). In a case where the treatment effects $\tau(L)$ and $\tau(R)$ are not distinguished, they are expressed as $\tau(l)$ (where 1 = L, R).

Next, the search unit 411 calculates a loss function before and after division by using the treatment effects $\tau(L)$ and $\tau(R)$ (step S1307). The loss function before the division is LossPre, and the loss function after the division is LossPost. First, the loss function LossPre before division is expressed by the following expression (3).

[Math. 2]

$$LossPre = N \cdot \tau \times W(x) \quad \cdots (3)$$

In the above expression (3), N on the right side is the number of samples of the search target group. In addition, $\tau$ on the right side is the treatment effect before division which is the first treatment effect. At the time of the first execution, the treatment effect $\tau$ in the parent node is used. After the second time of the loop, the treatment effect $\tau(l)$ after the previous division becomes the treatment effect $\tau$ before the division.

Further, x is the search target predictive factor specified in step S1305 among the explanatory variables 501 (x1, x2,..., xi,..., xn). W(x) is the weight 502 of the search target predictive factor.

In addition, in a case where the analysis target group is divided into the training data and the verification data in step S1302, the loss function LossPre before the division has a penalty term due to dispersion added to the above expression (3) and becomes as the following expression (4).

[Math. 3]

$$LossPre = N \cdot \tau \times W(x) - \left(1 + \frac{N_{train}}{N_{est}}\right) * \left(\frac{S_{T=1}^2}{p} + \frac{S_{T=0}^2}{1-p}\right)$$

$$\cdots (4)$$

$N_{train}$ on the right side of the above expression (4) is the number of samples of the training data, that is, the number of samples N of the search target group. $N_{est}$ is the number of samples of the verification data. $S_{T=1}$ is a variance of samples belonging to the treatment selection T = 1 in the search target group, and $S_{T=0}$ is a variance of samples belonging to the treatment selection T = 0 in the search target group. In addition, p is a ratio of the number of samples belonging to the treatment selection T = 1 in the search target group.

In addition, the entire right side of the above expressions (3) and (4) may be normalized by being divided by the number of samples N of the search target group.

Next, the loss function LossPost after the division is expressed by the following expression (5). The loss function LossPost after the division is a loss function that maximizes each estimated treatment effect $\tau(l)$.

[Math. 4]

$$LossPost = \sum_{l}^{L,R} N_l \cdot \tau_l \times W(x) \quad \cdots (5)$$

In the above expression (5), N(l) on the right side is the number of samples of the subtype 1. If the entire right side of the above expressions (3) and (4) is normalized by being divided by the number of samples N of the search target group, the entire right side of the above expression (5) may be normalized by being divided by the number of samples (total number of samples of subtypes L, R) of the search target group. In addition, val is a threshold for delimiting a range of the factor x. Instead of using val, W(x) may be used.

Next, the search unit 411 calculates a difference Gain between the loss functions LossPre and LossPost before and after the division (step S1308). The difference Gain is an index indicating whether the loss function LossPost has been improved by division.

$$Gain = LossPost - LossPre \cdots (6)$$

Next, the search unit 411 determines whether or not the current difference Gain is larger than the held difference Gain (step S1309). The held difference Gain is the difference Gain held in step S1310 of the previous loop and becomes a target value. However, at the time of the first execution, there is no held difference Gain, and thus, 0 is used as the initial value of the held difference Gain.

In a case where the current difference Gain is larger than the held difference Gain (step S1309: Yes), the search unit 411 updates the loss function LossPre before division applied this time with the loss function LossPost to obtain a new loss function LossPre before division, updates the held difference Gain with the current difference Gain and acquires branch conditions when the division into two is executed in step S1305. In this manner, the branch conditions are searched for. Then, the processing proceeds to step S1311.

On the other hand, in a case where the current difference Gain is not larger than the held difference Gain (step S1309: No), the processing proceeds to step S1311 without the search unit 411 updating the loss function LossPre before division and updating the held difference Gain.

Next, the search unit 411 determines whether or not division into two of the search target group (step S1305) satisfies end conditions (step S1311). The end conditions are, for example, a case where the explanatory variable 501 selectable as the search target is not left. In a case where the division into two of the search target group (step S1305) does not satisfy the end conditions (step S1305: No), that is, in a case where the explanatory variable 501 selectable as the search target is left, the processing returns to step S1304. In this case, the search unit 411 sets each of the subtypes L and R determined to be larger than the previous difference in step S1309 as the next search target group.

On the other hand, in a case where the end conditions are satisfied (step S1311: Yes), that is, in a case where the explanatory variable 501 selectable as the search target is not left, one causal tree is created, the search unit 411 stores the created causal tree, and the processing proceeds to step S1312.

Next, the search unit 411 determines whether or not end conditions of creation of the causal tree are satisfied (step S1312). The end conditions are, for example, a threshold of the number of causal trees. In a case where the end conditions are not satisfied (step S1312: No) (in a case where the number of created causal trees has not reached the threshold), the processing returns to step S1303, and the search unit 411 re-creates the factor list.

On the other hand, in a case where the end conditions are satisfied (step S1312: Yes), the search unit 411 outputs the created causal trees, and the processing proceeds to step S1203. As a result, the causal trees of the number corresponding to the threshold set in step S1312 are created. A node having a branch destination node in the node group constituting the causal tree includes the predictive factor and the division threshold used when division into groups are performed at the node.

<Simulation results>

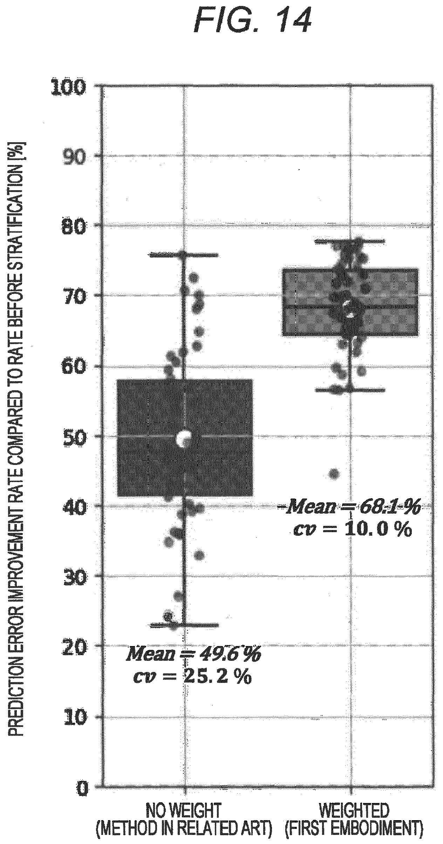

Next, simulation results of the first embodiment will be described with reference to Fig. 14.

Fig. 14 is a box-and-whisker diagram illustrating a prediction error improvement rate compared to the rate before division in the method in related art and in the first embodiment. The method in related art is a method of calculating the prediction error improvement rate by an expression obtained by removing W(x) from the above expressions (3) and (5).

$$Y_j = \eta(x_j) + T_j \cdot \tau(x_j) \cdots (7)$$

The expression (7) is an expression for calculating an outcome. A subscript j is the patient ID 601. $Y_j$ on the left side is the outcome of the patient with the value of the patient ID 601 of j (hereinafter, a patient j). $\eta(x_j)$ is an effect not related to treatment by the prognostic factor $x_j$ of the patient j. $T_j$ is the treatment selection T (= 0 or 1) for the patient j. $\tau(x_j)$ is a treatment effect by the predictive factor $x_j$.

Here, $\eta(x_j)$ is expressed by the following expression (8).

[Math. 5]

$$\eta(x_j) = 0.5 \left( \sum_{j=1}^{j=4} x_j \right) + \sum_{j=5}^{j=8} x_j \cdots (8)$$

In addition, $\tau(x_j)$ is expressed by the following expression (9).

[Math. 6]

$$\tau(x_j) = 3 \left( \sum\nolimits_{j=1}^{j=2} x_j \right) \quad \cdots (9)$$

The above expressions (8) and (9) are expressions indicating a data generation method by simulation, and table data similar to Fig. 7 is created. The number of samples N of the patient j was set to N = 1000, and the treatment selection $T_j$ of the patient j was set to random. Here, it is assumed that the values of the weights 502 of the factors x1 and x2 among the factors x1 to x8 are much larger than those of the other factors x3 to x8.

In this simulation, the prediction error reduction rates before and after division were calculated using a root mean square error (RMSE) as evaluation of accuracy. In the first embodiment, weighting is performed, and thus, it can be confirmed that the prediction error improvement rate is improved and a coefficient of variation (CV) is remarkably reduced.

Second embodiment

Next, the second embodiment will be described. The first embodiment has been described on the assumption that the weight table 430 exists, but the second embodiment is an example in which the analysis device 300 generates the weight table 430. In other words, in the second embodiment, the analysis device 300 generates the weight table 430 with reference to the patient data table 420 by the generation unit 400. In the second embodiment, a difference from the first embodiment will be mainly described, and description of common portions with the first embodiment will be omitted.

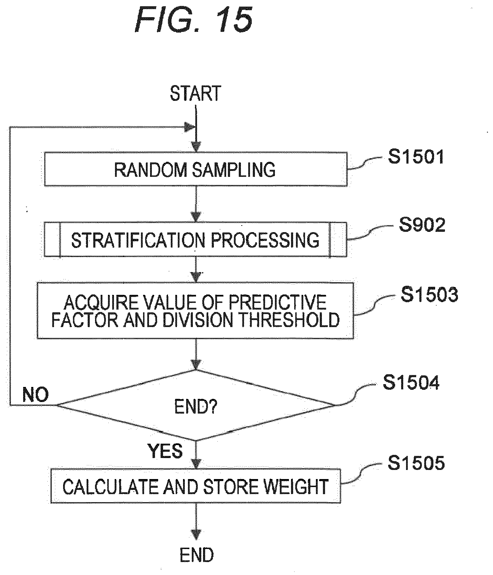

Fig. 15 is a flowchart indicating an example of processing procedure of generating the weight table 430 by the generation unit 400 according to the second embodiment. The generation unit 400 randomly samples entries defining patient data from the patient data table 420 (step S1501) . The number of samples is arbitrarily set, for example, 50% or 70% of all samples of the patient data table 420. In addition, the generation unit 400 may use samples that has not been sampled as verification data.

Next, the generation unit 400 outputs the sample group sampled in step S1501 to the stratification unit 402 and calls and executes the stratification processing (step S902) indicated in Fig. 12 from the stratification unit 402 (step S902).

Next, the generation unit 400 acquires the value of the explanatory variable 501 and the division threshold thereof for each explanatory variable 501 used for division from each branch group that is the stratification result by the stratification processing (step S902) (step S1503).

Then, the generation unit 400 determines whether or not end conditions are satisfied (step S1504) . Specifically, the end conditions are, for example, a case where the number of executions of steps S1501 to S1503 reaches a predetermined number of times. In a case where the end conditions are not satisfied (step S1504:No), that is, in a case where the number of executions of steps S1501 to S1503 has not reached the predetermined number of times, the processing returns to step S1501. On the other hand, in a case where the end conditions are satisfied (step S1504: Yes), that is, in a case where the number of executions of steps S1501 to S1503 reaches the predetermined number of times, the weight 502 is calculated for each explanatory variable 501 and stored in the weight table 430 (step S1505).

Specifically, for example, the generation unit 400 calculates a statistic of the value of the explanatory variable 501 and the division threshold for each explanatory variable 501 and sets the calculated value as the weight 502. More specifically, for example, a difference between the maximum value and the division threshold among the values of the explanatory variable 501 may be set as the weight 502, a difference between the median value and the division threshold among the values of the explanatory variable 501 may be set as the weight 502, a difference between the mode value and the division threshold among the values of the explanatory variable 501 may be set as the weight 502, or a difference between the average value and the division threshold of the values of the explanatory variable 501 may be set as the weight 502. In addition, the number of occurrences of the value of the explanatory variable 501 may be set as the weight 502.

In this manner, the analysis device 300 automatically learns the weight. Thus, the predictive factor to be used as the branch conditions can have a larger weight 502, and the estimation accuracy of the treatment effect can be improved.

Note that, the above-described stratification processing (step S902) is also applied to Fig. 9, and thus, in a case where the stratification processing (step S902) is executed in Fig. 9, the generation unit 400 may update the weight table 430 using the stratification result. As a result, the more analysis is performed by the analysis device 300, reliability

13

of the weight table 430 becomes higher, and estimation accuracy of the treatment effect becomes higher.

Furthermore, in the first embodiment, the arbitrarily, created weight table 430 is applied. However, in the second embodiment, a computer having the generation unit 400 other than the analysis device 300 may generate the weight table 430 in the generation processing according to the second embodiment, and the analysis device 300 may acquire the weight table 430 from the computer.

Third embodiment

Next, the third embodiment will be described. The first embodiment has been described on the assumption that the weight table 430 exists, but the third embodiment is an example in which the analysis device 300 generates the weight table 430. In other words, in the third embodiment, the analysis device 300 generates the weight table 430 with reference to a medical literature database such as PubMed by the generation unit 400. In the third embodiment, a difference from the first embodiment will be mainly described, and description of common portions with the first embodiment will be omitted.

Specifically, for example, the analysis device 300 causes the generation unit 400 to execute abstract search on the medical literature database, perform statistical processing on an appearance rate of the related word/phrase and set the statistical processing result as the weight 502 of the explanatory variable 501. In this manner, the analysis device 300 automatically learns medical knowledge.

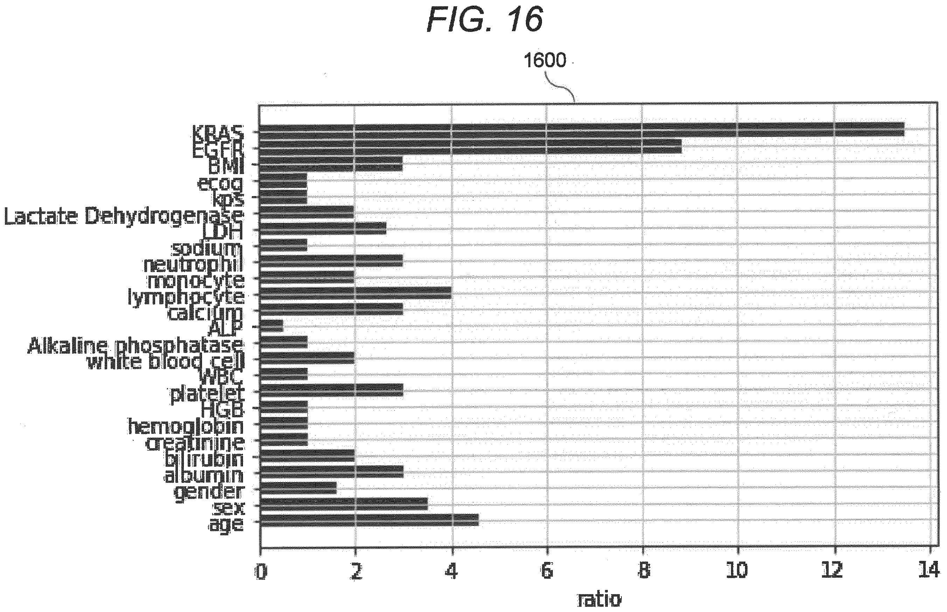

Fig. 16 is a histogram indicating search results from the medical literature database. A vertical axis of the histogram 1600 represents a column of factors included in sentences searched by a search keyword. For example, a name of a risk factor is used as the search keyword. In addition, the search keyword may include a conjunction related to an outcome such as "cause" or "relate".

A horizontal axis in Fig. 16 represents the weight 502 of the factor. The generation unit 400 calculates the value of the weight 502 so that the value becomes higher as the number of appearances of the search keyword or the number of sentences searched by the search keyword in the sentences searched by the search keyword is larger. However, if a negative word such as "not" is included in the sentences searched by the search keyword, the generation unit 400 performs calculation so as not to increase or to decrease the value of the weight 502.

The generation unit 400 excludes factors in which the value of the weight 502 is equal to or less than a predetermined threshold or the top $k + 1$ or less and stores the values of the weight 502 larger than the predetermined threshold or the top $k$-th factors in the weight table 430 together with the weight 502 as the explanatory variable 501.

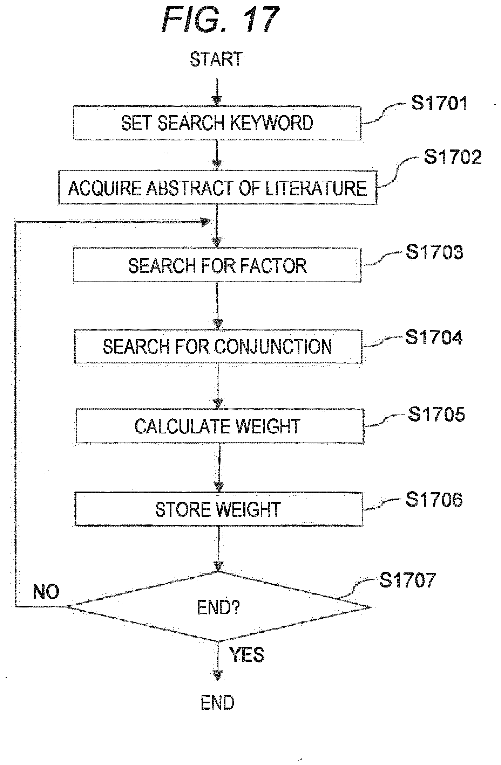

Fig. 17 is a flowchart indicating an example of processing procedure of generating the weight table 430 according to the third embodiment. The generation unit 400 sets the search keyword through user operation (step S1701). Next, the generation unit 400 transmits the search keyword to the medical literature database, searches for an abstract of each literature in the medical literature database and acquires an abstract of literature corresponding to the search keyword from the medical literature database (step S1702).

Next, the generation unit 400 searches the abstract acquired in step S1702 with the factor included in the search keyword and extracts sentences including the factor (step S1703).

Next, the generation unit 400 searches the sentences extracted in step S1703 for a conjunction related to the outcome (for example, "cause" or "relate") and increments a positive relationship count Cpos for the sentences including the conjunction. The positive relationship count Cpos is an evaluation value related to a sentence in which the relationship between the factor and the conjunction indicates positive, and the higher the count value, the larger the weight 502. On the other hand, in a case where a negative word such as "not" is included in the sentences searched as the conjunction related to the outcome, the generation unit 400 increments a negative relationship count Cneg.

Next, the generation unit 400 calculates the weight 502 for each factor (step S1705). The weight 502(w) is calculated by, for example, the following expression (10).

$$w = Cpos/Cneg \quad \cdots \quad (10)$$

Note that, if the negative relationship count Cneg of the denominator is not counted even once, Cneg = 0 and the calculation becomes impossible. Thus, expression (1) may be corrected so that the denominator of expression (10) does not become 0 even in a case where Cneg = 0.

Next, the generation unit 400 stores the calculated weight 502 in the weight table 430 (step S1706).

Then, the generation unit 400 determines whether or not end conditions are satisfied (step S1704). Specifically, the end conditions are, for example, a case where the weights 502 have been calculated for all the factors searched in step S1703. If there is a factor for which the weight 502 has not been calculated (step S1707: No), the processing returns to step S1703. On the other hand, if there is no factor for which the weight 502 has not been calculated (step S1707: Yes), the generation unit 400 ends the processing of an example.

In this manner, the analysis device 300 automatically learns medical knowledge as the weight. Thus, the factor searched from the medical literature database has a larger weight 502, and in a case where a factor having a medical basis from the medical literature is set as the predictive factor, the estimation accuracy of the treatment effect can be improved.

Note that, in the third embodiment, the abstract of the medical literature is set as the search target, and thus, the processing of generating the weight table 430 can be made faster than in a case where the medical literature itself is set as the search target. On the other hand, the generation unit 400 may use the medical literature itself as the search target. As a result, reliability of the weight 502 is improved as compared with a case where the abstract of the medical literature is used as the search target, and estimation accuracy of the treatment effect is improved.

Further, in the first embodiment, the arbitrarily created weight table 430 is applied. However, in the first embodiment, a computer having the generation unit 400 other than the analysis device 300 may generate the weight table 430 in the generation processing according to the third embodiment, and the analysis device 300 may acquire the weight table 430 from the computer.

Fourth embodiment

Next, the fourth embodiment will be described. The fourth embodiment is an example in which the causal tree is relearned in the first to third embodiments. In the fourth embodiment, a difference from the first to third embodiments will be mainly described, and thus, description of common portions with the first to third embodiments will be omitted. Note that if the weight $w(x)$ is not adopted (the value of the weight $w(x)$ of the above expression (1B) is 1), the example becomes a relearning example based on the above expression (1A), and if the weight $w(x)$ is adopted, the example becomes a relearning example based on the above expression (1B).

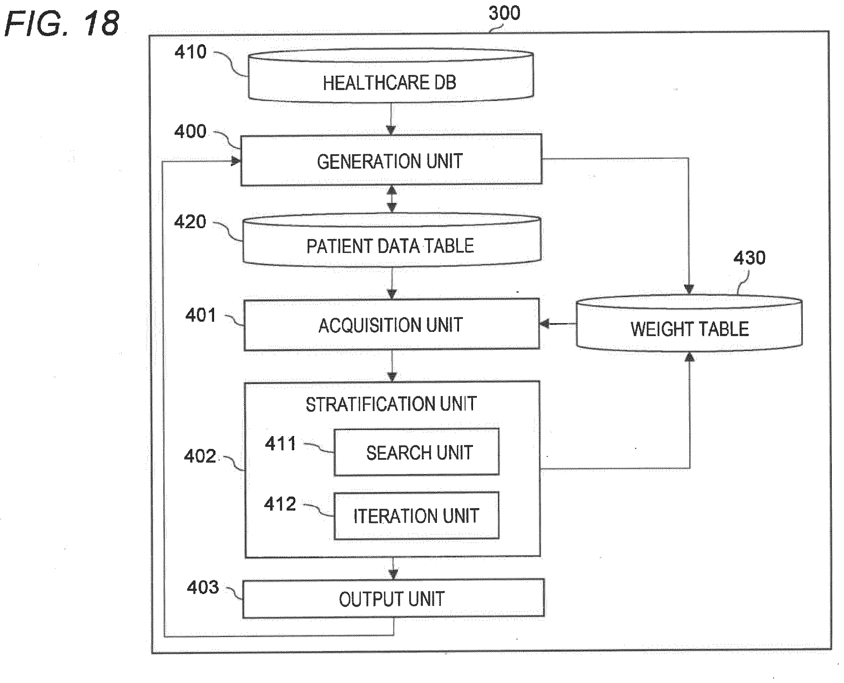

Fig. 18 is a block diagram illustrating a functional configuration example of the analysis device 300 according to the fourth embodiment. The analysis device 300 generates combined branch conditions by extracting and combining branch conditions from the causal tree 1000 output as the stratification result by the output unit 403 by the generation unit 400 and relearns the causal tree using the combined branch conditions. The search unit 411 generates a new causal tree by performing binary search of the combined branch conditions.

In the example of the causal tree 1000 of Fig 10, the branch conditions are $x1 > 0$, $x2 > 0$, and $x3 > 0$. The combined branch conditions are a combination of two or more branch conditions. In the example of the causal tree 1000 of Fig. 10, the combined branch conditions are $x1 > 0$ & $x2 > 0$, $x2 > 0$ & $x3 > 0$ and $x1 > 0$ & $x2 > 0$ & $x3 > 0$. "&" means a logical product. The combination of the conditional branches can also be a logical sum.

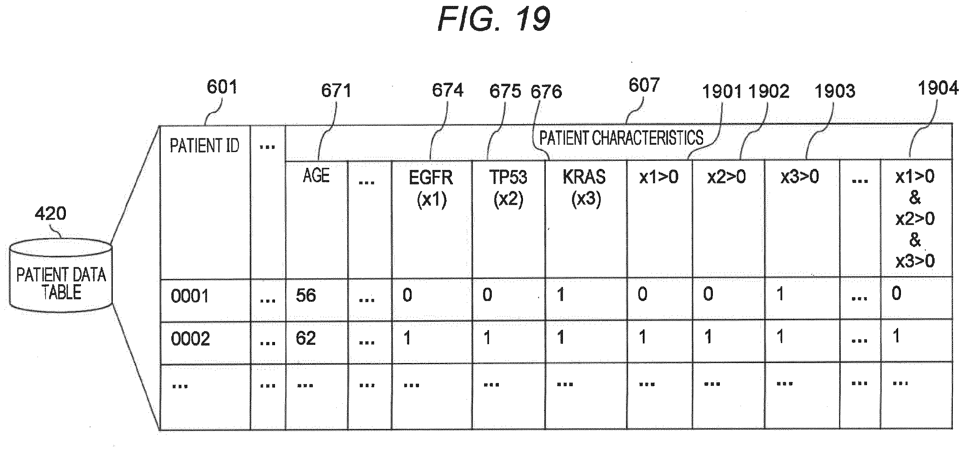

Fig. 19 is an explanatory diagram indicating an example of the patient data table 420 according to the fourth embodiment. Unlike the first embodiment, in the patient data table 420, branch conditions 1901 ($x1 > 0$), 1902 ($x2 > 0$), and 1903 ($x3 > 0$) and combined branch conditions 1904 ($x1 > 0$ & $x2 > 0$ & $x3 > 0$) are added to the patient characteristics 607. In addition to the combined branch conditions 1904, combined branch conditions of $x1 > 0$ & $x2 > 0$, $x2 > 0$ & $x3 > 0$, and other combined branch conditions including logical sum may also be added to the patient characteristics 607.

In the value of the branch conditions 1901 to 1903, "1" is stored in a case where the branch conditions 1901 to 1903 are satisfied, and "0" is stored in a case where the branch conditions are not satisfied. In the value of the branch conditions 1904, "1" is stored in a case where all of the branch conditions 1901 to 1903 are satisfied, and "0" is stored in a case where none of the branch conditions 1901 to 1903 are satisfied.

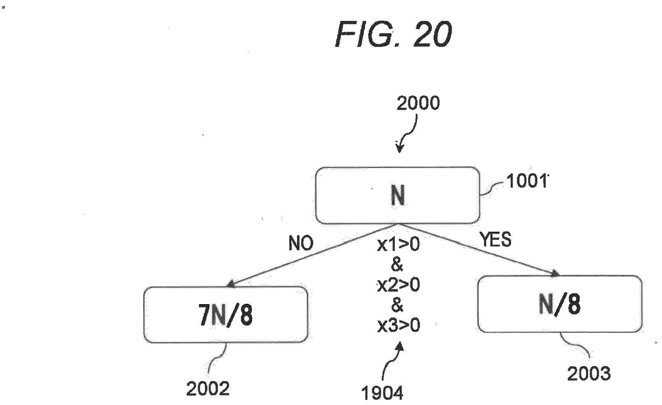

Fig. 20 is an explanatory diagram indicating an example of the causal tree generated by relearning. Fig. 20 indicates the causal tree 2000 searched for with the combined branch conditions 1904. The causal tree 2000 includes nodes 1001, 2002, and 2003.

The analysis target group indicated by the node 1001 is divided into a patient group (node 2003) satisfying the combined branch conditions 1904 and a patient group (node 2002) not satisfying the combined branch conditions 1904. The node 2003 satisfying the combined branch conditions 1904 is the same patient group as the node 1007 of the causal tree 1000 of Fig. 10. The node 2002 that does not satisfy the combined branch conditions 1904 is the same patient group as the patient group combining the nodes 1002, 1004, 1006 of the causal tree 1000 of Fig. 10.

Note that while Fig. 20 indicates the causal tree 2000 generated by searching with the combined branch conditions 1904, the combined branch conditions used for the search are not limited to the combined branch conditions 1904 and may be combined branch conditions of $x1 > 0$ & $x2 > 0$, $x2 > 0$ & $x3 > 0$, and other combined branch conditions including logical sum.

In addition, the analysis device 300 may perform search with unselected combined branch conditions or branch conditions also at and after the node 2002.

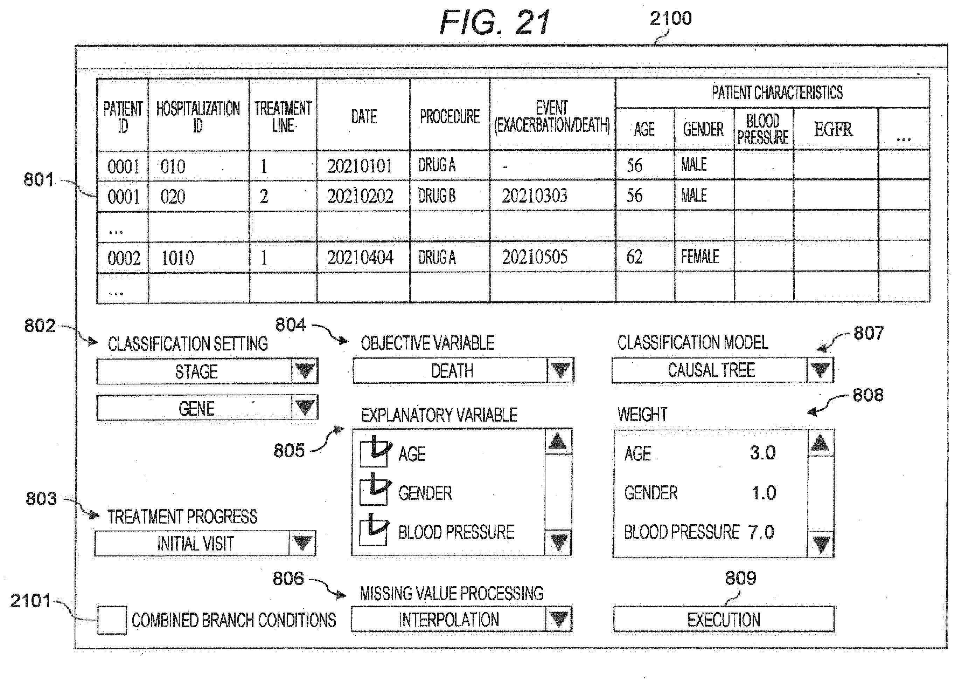

Fig. 21 is an explanatory diagram illustrating an example of an input screen of the analysis device 300 according to the first embodiment. The input screen 2100 includes a check box 2101 that accepts selection of whether or not to apply the combined branch conditions. If the check box 2101 is checked, the generation unit 400 generates combined branch conditions.

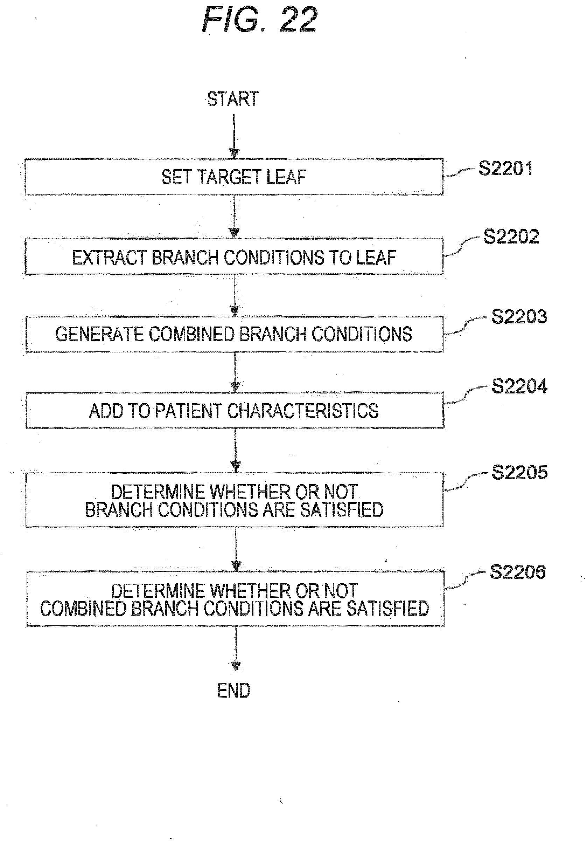

Fig. 22 is a flowchart indicating an example of processing procedure of generating the combined branch conditions by the generation unit 400 according to the fourth embodiment. The generation unit 400 sets a target leaf from the causal tree 1000 (step S2201). The leaf is a terminal node in a path that has passed through a plurality of branch conditions in the causal tree 1000. In the example of Fig. 10, the nodes are nodes 1004, 1006, and 1007. As described above, in a case where there are a plurality of target leaves, the generation unit 400 sets, for example, an end node of a path that satisfies all branch conditions, in this example, the node 1007. Furthermore, the generation unit 400 may randomly set the target leaf or may set a node selected through user operation as the target leaf.

The generation unit 400 extracts the branch conditions from the highest node 1001 to the leaf set in step S2201 (step S2202). In a case of the causal tree 1000 in Fig. 10, the branch conditions 1901 to 1903 are extracted.

The generation unit 400 generates combined branch conditions using the branch conditions extracted in step S2202 (step S2203). In a case of the causal tree 1000 in Fig. 10, the branch conditions 1904 are generated. In addition, the generation unit 400 may generate combined branch conditions of $x1 > 0$ & $x2 > 0$, $x2 > 0$ & $x3 > 0$, and other combined branch conditions including logical sum as well as the combined branch conditions 1904.

The generation unit 400 adds the branch conditions extracted in step S2202 and the combined branch conditions generated in step S2203 to the patient characteristics 607 of the patient data table 420 (step S2204).

The generation unit 400 determines whether or not the branch conditions are satisfied in each entry of the patient data table 420 and stores a value of the determination result (step S2205). In the example of Fig. 19, values of the respective determination results of the branch conditions 1901 are 1903 are stored. For example, for the entry of the patient with the patient ID 601 of "0001", "0" is stored as the value of the determination result of the branch conditions 1901 ($x1 > 0$) with reference to the value "0" of the EGFR 674, "0" is stored as the value of the determination result of the branch conditions 1902 ($x2 > 0$) with reference to the value "0" of the TP53 675, and "1" is stored as the value of the determination result of the branch conditions 1903 ($x3 > 0$) with reference to the value "1" of the KRAS 676.

The generation unit 400 determines whether or not the combined branch conditions are satisfied in each entry of the patient data table 420 and stores the value of the determination result (step S2206). In the example of Fig. 19, a value of the determination result of the combined branch conditions 1904 is stored. For example, for the entry of the patient with the patient ID 601 of "0001", "0" is stored as the logical product of the value "0" of the determination result of the branch conditions 1901 ($x1 > 0$), the value "0" of the determination result of the branch conditions 1902 ($x2 > 0$), and the value "1" of the determination result of the branch conditions 1903 ($x3 > 0$) .

Then, the analysis device 300 executes the processing indicated in Fig. 9 as in the first embodiment. The analysis device 300 acquires the combined branch conditions 1904 from the patient data table 420 by the acquisition unit 401 and executes the stratification processing (step S902). The analysis device 300 generates the causal tree 2000 in the branch condition search processing (step S1202) in the stratification processing (step S902).

Specifically, for example, the search unit 411 selects the combined branch conditions registered in the patient data table 420 in the creation of the factor list in step S1303 and creates the factor list. For example, the combined branch conditions 1904 are registered in the factor list.

Next, in the creation of the factor value list in step S1304, the search unit 411 extracts the values of the combined branch conditions in each entry registered in the factor list and registers the values in the factor value list. For example, values "0", "1",... of the combined branch conditions 1904 are registered in the factor value list.

After step S1305, the same processing as in the first embodiment is executed. As a result, the causal tree 2000 as indicated in Fig. 20 is created.

Comparing the causal trees 1000 and 2000, the causal tree 1000 has four stages, and the causal tree 2000 has two stages. If multi-stage learning is executed on the causal tree, the number of samples N (the number of patients) decreases as the causal tree becomes deeper, that is, the number of stages increases. This situation frequently occurs particularly in the medical field. The loss function to be used for learning is a function of the estimated treatment effect $\tau$, and the estimated treatment effect $\tau$ is calculated as an expected value, and thus, the accuracy decreases as the number of samples N decreases.

On the other hand, the number of stages of the causal tree 2000 is smaller than that of the causal tree 1000. Thus, the analysis device 300 can perform search with fewer branches and a combination of complicated branch conditions, so that it is possible to prevent decrease in the number of samples at the time of learning.

Note that, in the fourth embodiment, the analysis device 300 generates the causal tree 1000 and generates the causal tree 2000 using the causal tree 1000, but the analysis device 300 may acquire the causal trees 1000 and 2000 from the outside without generating the causal trees 1000 and 2000. Also, rather than extracting conditions from the causal tree 1000 as indicated in Fig. 22, the analysis device 300 may use the EGFR 674, the TP53 675, and the KRAS 676 in the patient characteristics 607 of the patient data table 420 to generate the branch conditions 1901 to 1903 and combined branch conditions 1904. In this case, the analysis device 300 acquires the combined branch conditions 1904 from the patient data table 420 in which the branch conditions 1901 to 1903 and the combined branch conditions 1904 are generated by the acquisition unit 401 and executes the stratification processing (step S902). The analysis device 300 generates the causal tree 2000 in the branch condition search processing (step S1202) in the stratification processing (step S902).

As described above, according to the analysis device 300 described above, it is possible to execute learning under complicated branch conditions based on the group of data to be analyzed that is the original population, so that it is possible to implement more accurate estimation by using the causal tree 2000 obtained by this learning.

In addition, according to the analysis device 300 described above, weighting is performed on predictive factors estimated from empirical knowledge and medical literature in advance, so that it is possible to improve classification accuracy in a case of stratifying patients by factors contributing to the treatment effect. It is therefore possible to improve estimation accuracy of the treatment effect and implement more correct patient stratification.

In other words, the analysis device 300 can directly classify the patients into subtypes on the basis of the estimated treatment effect according to the patient characteristics. Thus, stratified patient groups are classified as subtypes with different treatment effects and are expected to contribute to optimal treatment selection that suits individual patient characteristics. It is therefore possible to specify a subtype for which a treatment effect by a certain drug can be expected.

Note that, in the first to fourth embodiments described above, the patient data has been described as an example of the data to be analyzed, but the data to be analyzed is not limited to the patient data as long as the causal tree 2000 can be generated.

Note that the present invention is not limited to the above-described embodiments and includes various modifications and equivalent configurations within the spirit of the appended claims. For example, the above-described embodiments have been described in detail for easy understanding of the present invention, and the present invention is not necessarily limited to those having all the described components. Further, part of the components of one embodiment may be replaced with the components of another embodiment. In addition, the component of another embodiment may be added to the component of one embodiment. In addition, part of the components of each embodiment may be added, deleted, or replaced with another components.

In addition, part or all of each of the above-described components, functions, processing units, processing means, and the like, may be implemented by hardware, for example, by designing with an integrated circuit or may be implemented by software, by a processor interpreting and executing a program for implementing each function.

Information such as a program, a table, and a file for implementing each function can be stored in a storage device such as a memory, a hard disk, and a solid state drive (SSD), or a recording medium such as an integrated circuit (IC) card, an SD card, and a digital versatile disc (DVD) .

In addition, control lines and information lines indicate what is considered to be necessary for the description, and not all the control lines and information lines necessary for implementation are necessarily indicated. In practice, it may be considered that almost all the components are connected to each other.

**Claims**

1. An analysis device comprising:

   an acquisition unit that acquires, for a plurality of pieces of data to be analyzed having a value of each factor in a factor group, combined branch conditions obtained by combining a plurality of branch conditions in the factor group and values of the combined branch conditions of each piece of data to be analyzed of the plurality of pieces of data to be analyzed; and
   a search unit that divides the plurality of pieces of data to be analyzed having the values of the combined branch conditions on a basis of the combined branch conditions and searches for a first decision tree.

2. The analysis device according to claim 1, wherein the acquisition unit acquires combined branch conditions including the two branch conditions combined by a logical product.

3. The analysis device according to claim 1, wherein the acquisition unit acquires combined branch conditions including the two branch conditions combined by a logical sum.

4. The analysis device according to claim 1, wherein the acquisition unit acquires, for the plurality of pieces of data to be analyzed, combined branch conditions obtained by combining a plurality of branch conditions in the factor group in a second decision tree and values of the combined branch conditions of each piece of data to be analyzed of the plurality of pieces of data to be analyzed.

5. The analysis device according to claim 4, further comprising a generation unit that generates the combined branch conditions and the values of the combined branch conditions,

wherein

the search unit generates a second decision tree by repeatedly executing selection processing of selecting the factor, division processing of dividing the plurality of pieces of data to be analyzed that are division targets on a basis of the factor selected by the selection processing, and setting processing of setting the plurality of pieces of data to be analyzed obtained by the division processing as new division targets and executing search processing of searching for the branch conditions for dividing the division targets by the division processing, and the generation unit generates, for the plurality of pieces of data to be analyzed, combined branch conditions obtained by combining a plurality of branch conditions in the factor group in the second decision tree obtained from the search unit.

6. The analysis device according to claim 5, further comprising a storage unit that stores a weight for each factor of the factor group,
wherein the search unit generates a second decision tree by repeatedly executing selection processing of selecting the factor and the weight, division processing of dividing the plurality of pieces of data to be analyzed that are division targets on a basis of the factor and the weight selected by the selection processing, and setting processing of setting the plurality of pieces of data to be analyzed obtained by the division processing as new division targets and executing search processing of searching for the branch conditions for dividing the division targets by the division processing.

7. The analysis device according to claim 5, wherein the data to be analyzed is patient data having a value of each factor in the factor group.

8. The analysis device according to claim 7, wherein

the patient data includes variables related to treatment selection that indicate whether or not a patient has selected treatment, and
in a case where a plurality of pieces of the patient data are set as the division targets by the setting processing, the search unit executes treatment effect calculation processing of calculating a first treatment effect related to the factor using the variables for the plurality of pieces of patient data and calculating a second treatment effect related to the factor using the variables for each of two patient data groups divided by the division processing, loss function calculation processing of calculating a loss function before division on a basis of the first treatment effect and the factor and calculating a loss function after division on a basis of the second treatment effect and the factor of each of the two patient data groups, and difference calculation processing of calculating a difference between the loss function before division and the loss function after division and searches for the branch conditions on a basis of the difference.

9. The analysis device according to claim 7, wherein

the patient data includes variables related to treatment selection that indicate whether or not a patient has selected treatment, and
the search unit executes treatment effect calculation processing of calculating a first treatment effect relating to the factors using the variables for a plurality of pieces of the patient data in a case where the plurality of pieces of patient data are set as the division targets by the setting processing and calculating a second treatment effect relating to the factor using the variables for each of two patient data groups divided by the division processing, loss function calculation processing of calculating a loss function before division on a basis of the first treatment effect, the factor, and the weight and calculating a loss function after division on a basis of the second treatment effect, the factor, and the weight of each of the two patient data groups, and difference calculation processing of calculating a difference between the loss function before division and the loss function after division and searches for the branch conditions on a basis of the difference.

10. The analysis device according to claim 8,
wherein in a case where the difference is larger than a target value, the search unit executes update processing of updating the loss function before the division with the loss function after the division and updating the target value with the difference.

11. The analysis device according to claim 8,
wherein

the search unit executes the search processing using the plurality of pieces of patient data as an analysis target group,

the analysis device comprising a stratification unit that performs stratification processing of temporarily dividing the analysis target group into a first branch group and a second branch group under the branch conditions on a basis of the factor and the weight and executing determination processing of determining whether or not the second treatment effect of any one of the first branch group and the second branch group has significantly changed on a basis of a comparison result between the first treatment effect of the analysis target group and the second treatment effect of the first branch group and a comparison result between the first treatment effect of the analysis target group and the second treatment effect of the second branch group, thereby dividing the analysis target group into the first branch group and the second branch group on a basis of a determination result by the determination processing.

12. The analysis device according to claim 6,
wherein the factor is a predictive factor reflecting sensitivity to treatment.

13. The analysis device according to claim 1,
wherein the first decision tree is a causal tree.

14. An analysis method to be executed by an analysis device including a processor that executes a program and a storage device that stores the program, the analysis method comprising:

the processor
acquiring, for a plurality of pieces of data to be analyzed having a value of each factor in a factor group, combined branch conditions obtained by combining a plurality of branch conditions in the factor group and values of the combined branch conditions of each piece of data to be analyzed of the plurality of pieces of data to be analyzed; and
dividing the plurality of pieces of data to be analyzed having the values of the combined branch conditions on a basis of the combined branch conditions and searching for a first decision tree.

15. An analysis program causing a processor to:

acquire, for a plurality of pieces of data to be analyzed having a value, of each factor in a factor group, combined branch conditions obtained by combining a plurality of branch conditions in the factor group and values of the combined branch conditions of each piece of data to be analyzed of the plurality of pieces of data to be analyzed; and
divide the plurality of pieces of data to be analyzed having the values of the combined branch conditions on a basis of the combined branch conditions and search for a first decision tree.

EP 4 404 106 A1

# FIG. 1

OBSERVATION VALUE
(LIFE OR DEATH, PROGRESSION-FREE PERIOD, TUMOR SIZE, etc.)

NOT DIRECTLY OBSERVABLE

OUTCOME=EFFECT NOT RELATED TO TREATMENT+TREATMENT EFFECT

> PROGNOSTIC FACTOR: FACTOR INDICATING INDEPENDENT PROGNOSIS REGARDLESS OF WHETHER OR NOT TREATMENT IS PERFORMED

PATIENT GROUP A
PATIENT GROUP B

(AGE low)

TREATMENT EFFECT $\tau$

THERE IS NO DIFFERENCE IN TREATMENT EFFECT

OUTCOME

(AGE high)

TREATMENT EFFECT $\tau$

TREATMENT IS NOT PERFORMED

TREATMENT IS PERFORMED

101

> PREDICTIVE FACTOR: FACTOR REFLECTING SENSITIVITY TO TREATMENT

PATIENT GROUP C
PATIENT GROUP D

(EGFR+)

THERE IS DIFFERENCE IN TREATMENT EFFECT

OUTCOME

GREAT TREATMENT EFFECT $\tau$

(EGFR-)

NO TREATMENT EFFECT $\tau$

TREATMENT IS NOT PERFORMED

TREATMENT IS PERFORMED

102

# FIG. 2

POPULATION

201 : PATIENTS BELONGING TO PROCEDURE GROUP

202 : PATIENTS BELONGING TO NON-PROCEDURE GROUP

(+)/(-) : OUTCOME (RESPONSE/NON-RESPONSE)

200

DIVIDE BY PATIENT CHARACTERISTICS

SUBTYPE L

SUBTYPE R

ESTIMATED TREATMENT EFFECT $\tau(L)$

ESTIMATED TREATMENT EFFECT $\tau(R)$

$$f = \sum_{l}^{L,R} \{N(l) \cdot \tau(l)^2\} \qquad \cdots (1A)$$

$$f = \sum_{l}^{L,R} \{N(l) \cdot \tau(l)^2\} \times w(x) \qquad \cdots (1B)$$

EP 4 404 106 A1

# FIG. 3

300

301

302

PROCESSOR

STORAGE
DEVICE

306

INPUT
DEVICE

OUTPUT
DEVICE

COMMUNICATION
IF

303

304

305

*FIG. 4*

EP 4 404 106 A1

# FIG. 5

| EXPLANATORY VARIABLE 501 | WEIGHT 502 |
|---|---|
| x1 | w(x1) |
| x2 | w(x2) |
| ⋮ | ⋮ |
| xi | w(xi) |
| ⋮ | ⋮ |
| xn | w(xn) |

430
WEIGHT TABLE

## FIG. 6

|  |  |  |  |  |  | PATIENT CHARACTERISTICS | | | | | |
|  | | | | | | | | | 607 | | |
| PATIENT ID (601) | HOSPITALIZATION ID (602) | TREATMENT LINE (603) | DATE (604) | PROCEDURE (605) | EVENT (EXACERBATION/ DEATH) (606) | AGE (671) | GENDER (672) | BLOOD PRESSURE (673) | EGFR (674) | TP53 (675) | KRAS (676) |
|---|---|---|---|---|---|---|---|---|---|---|---|
| 0001 | 010 | 1 | 20210101 | DRUG A | - | 56 | MALE | ... | ... | ... | ... |
| 0001 | 020 | 2 | 20210202 | DRUG B | 20210303 | 56 | MALE | ... | ... | ... | ... |
| ... | ... | ... | ... | ... | ... | ... | ... | ... | ... | ... | ... |
| 0002 | 1010 | 1 | 20210404 | DRUG A | 20210505 | 62 | FEMALE | ... | ... | ... | ... |
| ... | ... | ... | ... | ... | ... | ... | ... | ... | ... | ... | ... |

HEALTHCARE DB (410)

## FIG. 7

EP 4 404 106 A1

| PATIENT ID | SURVIVAL PERIOD | OUTCOME | TREATMENT SELECTION | AGE | GENDER | BLOOD PRESSURE | PATIENT CHARACTERISTICS | | |
|---|---|---|---|---|---|---|---|---|---|
| | | | | | | | EGFR (x1) | TP53 (x2) | KRAS (x3) |
| 0001 | 90 | 1 | 0 | 56 | MALE | ... | 0 | 0 | 1 |
| 0002 | 224 | 0 | 1 | 62 | FEMALE | ... | 1 | 1 | 1 |
| ... | ... | ... | ... | ... | ... | ... | ... | ... | ... |

420 PATIENT DATA TABLE

FIG. 8

800

| PATIENT ID | HOSPITALIZATION ID | TREATMENT LINE | DATE | PROCEDURE | EVENT (EXACERBATION/DEATH) | PATIENT CHARACTERISTICS | | | | |
|---|---|---|---|---|---|---|---|---|---|---|
| | | | | | | AGE | GENDER | BLOOD PRESSURE | EGFR | ... |
| 0001 | 010 | 1 | 20210101 | DRUG A | - | 56 | MALE | | | |
| 0001 | 020 | 2 | 20210202 | DRUG B | 20210303 | 56 | MALE | | | |
| ... | | | | | | | | | | |
| 0002 | 1010 | 1 | 20210404 | DRUG A | 20210505 | 62 | FEMALE | | | |
| ... | | | | | | | | | | |

801

802 CLASSIFICATION SETTING

| STAGE | ▼ |

| GENE | ▼ |

803 TREATMENT PROGRESS

| INITIAL VISIT | ▼ |

804 OBJECTIVE VARIABLE

| DEATH | ▼ |

805 EXPLANATORY VARIABLE

☑ AGE
☑ GENDER
☑ BLOOD PRESSURE

806 MISSING VALUE PROCESSING

| INTERPOLATION | ▼ |

807 CLASSIFICATION MODEL

| CAUSAL TREE | ▼ |

808 WEIGHT

AGE           3.0
GENDER        1.0
BLOOD PRESSURE 7.0

809

| EXECUTION |

EP 4 404 106 A1

# FIG. 9

START

ACQUIRE PATIENT INFORMATION — S901

STRATIFICATION PROCESSING — S902

OUTPUT STRATIFICATION RESULT — S903

END

EP 4 404 106 A1

FIG. 10

FIRST BRANCH GROUP

SECOND BRANCH GROUP

THIRD BRANCH GROUP

FOURTH BRANCH GROUP

FIFTH BRANCH GROUP

SIXTH BRANCH GROUP

RESPONSE GROUP

1000 — N

1001

x1>0 YES → N/2 (1001)
NO → N/2 (1002)

x2>0 YES → N/4 (1003)
NO → N/4 (1004)

x3>0 YES → N/8 (1005 → 1007)
NO → N/8 (1006)

29

## FIG. 11

# FIG. 12

# FIG. 13

S1202 — BRANCH CONDITION SEARCH PROCESSING

S1201

S1301 — READ WEIGHT

S1302 — ACQUIRE SEARCH TARGET GROUP

S1303 — CREATE FACTOR LIST

S1304 — CREATE FACTOR VALUE LIST

S1305 — DIVIDE SEARCH TARGET GROUP INTO TWO

S1306 — CALCULATE TREATMENT EFFECT

S1307 — CALCULATE LOSS FUNCTION

S1308 — CALCULATE DIFFERENCE IN LOSS FUNCTION BEFORE AND AFTER DIVISION

S1309 — IS DIFFERENCE LARGER THAN PREVIOUS DIFFERENCE?

YES

S1310 — UPDATE LOSS FUNCTION

NO

S1311 — HAS DIVISION INTO TWO ENDED?

NO

YES

S1312 — HAS CREATION OF CAUSAL TREE ENDED?

NO

YES

S1203

# FIG. 14

# FIG. 15

START

RANDOM SAMPLING —— S1501

STRATIFICATION PROCESSING —— S902

ACQUIRE VALUE OF PREDICTIVE FACTOR AND DIVISION THRESHOLD —— S1503

NO ◀— END? —— S1504

YES

CALCULATE AND STORE WEIGHT —— S1505

END

EP 4 404 106 A1

## FIG. 16

1600

# FIG. 17

START

SET SEARCH KEYWORD — S1701

ACQUIRE ABSTRACT OF LITERATURE — S1702

SEARCH FOR FACTOR — S1703

SEARCH FOR CONJUNCTION — S1704

CALCULATE WEIGHT — S1705

STORE WEIGHT — S1706

NO ── END? — S1707

YES

END

FIG. 18

# FIG. 19

| PATIENT ID | ... | AGE | ... | EGFR (x1) | TP53 (x2) | KRAS (x3) | x1>0 | x2>0 | x3>0 | ... | x1>0 & x2>0 & x3>0 |
|---|---|---|---|---|---|---|---|---|---|---|---|
| 0001 | ... | 56 | ... | 0 | 0 | 1 | 0 | 0 | 1 | ... | 0 |
| 0002 | ... | 62 | ... | 1 | 1 | 1 | 1 | 1 | 1 | ... | 1 |
| ... | ... | ... | ... | ... | ... | ... | ... | ... | ... | ... | ... |

PATIENT DATA TABLE (420)

PATIENT CHARACTERISTICS

## FIG. 20

2000

N — 1001

NO

x1>0
&
x2>0
&
x3>0

1904

YES

7N/8

2002

N/8

2003

*FIG. 21*　　2100

| PATIENT ID | HOSPITALIZATION ID | TREATMENT LINE | DATE | PROCEDURE | EVENT (EXACERBATION/DEATH) | PATIENT CHARACTERISTICS | | | | |
|---|---|---|---|---|---|---|---|---|---|---|
| | | | | | | AGE | GENDER | BLOOD PRESSURE | EGFR | ... |
| 0001 | 010 | 1 | 20210101 | DRUG A | - | 56 | MALE | | | |
| 0001 | 020 | 2 | 20210202 | DRUG B | 20210303 | 56 | MALE | | | |
| ... | | | | | | | | | | |
| 0002 | 1010 | 1 | 20210404 | DRUG A | 20210505 | 62 | FEMALE | | | |
| ... | | | | | | | | | | |

801

802 → CLASSIFICATION SETTING

| STAGE | ▼ |
|---|---|

| GENE | ▼ |
|---|---|

804 → OBJECTIVE VARIABLE

| DEATH | ▼ |
|---|---|

805 → EXPLANATORY VARIABLE

- ☑ AGE
- ☑ GENDER
- ☑ BLOOD PRESSURE

807 → CLASSIFICATION MODEL

| CAUSAL TREE | ▼ |
|---|---|

808

WEIGHT

| AGE | 3.0 |
|---|---|
| GENDER | 1.0 |
| BLOOD PRESSURE | 7.0 |

803 → TREATMENT PROGRESS

| INITIAL VISIT | ▼ |
|---|---|

2101 → ☐ COMBINED BRANCH CONDITIONS

806 → MISSING VALUE PROCESSING

| INTERPOLATION | ▼ |
|---|---|

809

| EXECUTION |
|---|

# FIG. 22

START

SET TARGET LEAF — S2201

EXTRACT BRANCH CONDITIONS TO LEAF — S2202

GENERATE COMBINED BRANCH CONDITIONS — S2203

ADD TO PATIENT CHARACTERISTICS — S2204

DETERMINE WHETHER OR NOT
BRANCH CONDITIONS ARE SATISFIED — S2205

DETERMINE WHETHER OR NOT
COMBINED BRANCH CONDITIONS ARE SATISFIED — S2206

END

Europäisches
Patentamt

European
Patent Office

Office européen
des brevets

# EUROPEAN SEARCH REPORT

**Application Number**

EP 23 21 8500

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PREETAM NANDY ET AL: "Generalized Causal Tree for Uplift Modeling", ARXIV.ORG, CORNELL UNIVERSITY LIBRARY, 201 OLIN LIBRARY CORNELL UNIVERSITY ITHACA, NY 14853, 4 February 2022 (2022-02-04), XP091151031, * abstract * * sections 2, 3, A and B * | 1-15 | INV. G06N5/01 G06N5/045 G06N20/00 G16H50/20 |
| X | JP 3 897169 B2 (FUJI ELECTRIC HOLDINGS) 22 March 2007 (2007-03-22) * figures 3, 4, 9 * * paragraphs [0033] - [0089] * | 1-15 | |
| X | US 2020/302318 A1 (ORACLE INT CORP [US]) 24 September 2020 (2020-09-24) * paragraphs [0026] - [0047], [0056] * * paragraph [0082] * * paragraphs [0111] - [0116] * * figure 1 * | 1-15 | |
| A | JP 6 901308 B2 (HITACHI LTD) 14 July 2021 (2021-07-14) * paragraphs [[125]] - [[133]]; figures 18, 19 * | 6,9 | TECHNICAL FIELDS SEARCHED (IPC)  G06N G16H |
| A | XINYUAN CHEN ET AL: "A Bayesian Machine Learning Approach for Estimating Heterogeneous Survivor Causal Effects: Applications to a Critical Care Trial", ARXIV.ORG, CORNELL UNIVERSITY LIBRARY, 201 OLIN LIBRARY CORNELL UNIVERSITY ITHACA, NY 14853, 10 January 2023 (2023-01-10), XP091411263, * sections 2, 3 and 4.4 * | 11 | |

The present search report has been drawn up for all claims

| Place of search | Date of completion of the search | Examiner |
|---|---|---|
| The Hague | 11 June 2024 | Millet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 8500

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2024

| Patent document cited in search report | | Publication date | Patent family member(s) | | Publication date |
|---|---|---|---|---|---|
| JP 3897169 | B2 | 22-03-2007 | JP 3897169 B2 | | 22-03-2007 |
| | | | JP 2004157814 A | | 03-06-2004 |
| US 2020302318 | A1 | 24-09-2020 | NONE | | |
| JP 6901308 | B2 | 14-07-2021 | JP 6901308 B2 | | 14-07-2021 |
| | | | JP 2018180993 A | | 15-11-2018 |

For more details about this annex : see Official Journal of the European Patent Office, No. 12/82

## REFERENCES CITED IN THE DESCRIPTION

**Patent documents cited in the description**

- JP 2023006486 A

**Non-patent literature cited in the description**

- **ATHEY, SUSAN et al.** Recursive partitioning for heterogeneous causal effects. *Proceedings of the National Academy of Sciences,* 2016, vol. 113 (27), 7353-7360